(19) Europäisches Patentamt
European Patent Office
Office européen des brevets

(11) Veröffentlichungsnummer : **0 075 117**
**B1**

(12) # EUROPÄISCHE PATENTSCHRIFT

(45) Veröffentlichungstag der Patentschrift : **13.03.85**

(51) Int. Cl.⁴ : **C 07 D285/00**, A 01 N 43/72

(21) Anmeldenummer : **82107611.4**

(22) Anmeldetag : **20.08.82**

(54) **2H-1,2,4,6-Thiatriazin-1,1-dioxide, Verfahren zu ihrer Herstellung und ihre Verwendung zur Bekämpfung unerwünschten Pflanzenwuchses.**

(30) Priorität : **28.08.81 DE 3134145**

(43) Veröffentlichungstag der Anmeldung : **30.03.83 Patentblatt 83/13**

(45) Bekanntmachung des Hinweises auf die Patenterteilung : **13.03.85 Patentblatt 85/11**

(84) Benannte Vertragsstaaten : **AT BE CH DE FR GB IT LI NL**

(56) Entgegenhaltungen : **EP-A- 0 039 426**

(73) Patentinhaber : **BASF Aktiengesellschaft**
**Carl-Bosch-Strasse 38**
**D-6700 Ludwigshafen (DE)**

(72) Erfinder : **Hamprecht, Gerhard, Dr.**
**Rote-Turm-Strasse 28**
**D-6940 Weinheim (DE)**
Erfinder : **Parg, Adolf, Dr.**
**Paray-le-Monial-Strasse 8**
**D-6702 Bad Duerkheim (DE)**
Erfinder : **Wuerzer, Bruno, Dr. Dipl.-Landwirt**
**Ruedigerstrasse 13**
**D-6701 Otterstadt (DE)**

**EP 0 075 117 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 2H-1,2,4,6-Thiatriazin-1,1-dioxide, Verfahren zu ihrer Herstellung, Herbizide, die diese Verbindungen als Wirkstoffe enthalten und ihre Verwendung zur Bekämpfung unerwünschten Pflanzenwuchses.

Es ist bekannt, daß substituierte 6H-1,2,4,6-Thiatriazin-(5)-on-1,1-dioxidderivate eine herbizide Wirkung haben (DE-OS 25 08 832, 29 33 889).

Es wurde gefunden, daß 2H-1,2,4,6-Thiatriazin-1,1-dioxide der Formel

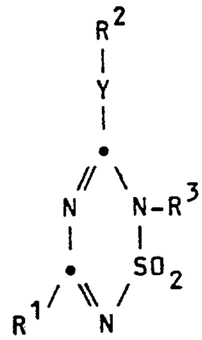

(I)

in der

$R^1$ Wasserstoff, einen gesättigten oder ungesättigten, unverzweigten oder verzweigten aliphatischen Rest mit bis zu 10 Kohlenstoffatomen, einen durch Halogen, Alkoxy oder Alkylmercapto mit 1 bis 4 Kohlenstoffatomen substituierten gesättigten, unverzweigten oder verzweigten aliphatischen Rest mit bis zu 10 Kohlenstoffatomen, einen Alkyl- oder Dialkylaminorest mit 1 bis 6 Kohlenstoffatomen in einer Alkylgruppe, einen Cycloalkylrest mit 3 bis 7 Kohlenstoffatomen, einen gegebenenfalls durch Halogen, Alkyl oder Alkoxy mit 1 bis 4 Kohlenstoffatomen substituierten Phenylrest oder einen gegebenenfalls durch Halogen substituierten Benzylrest,

$R^2$ einen gesättigten oder ungesättigten, unverzweigten oder verzweigten aliphatischen Rest mit bis zu 10 Kohlenstoffatomen, einen durch Halogen, Alkoxy oder Alkylmercapto mit 1 bis 4 Kohlenstoffatomen oder Alkanoyl mit 2 bis 8 Kohlenstoffatomen substituierten gesättigten oder ungesättigten, unverzweigten oder verzweigten aliphatischen Rest mit bis zu 10 Kohlenstoffatomen, einen Cycloalkylrest mit 3 bis 7 Kohlenstoffatomen, einen Alkoxycarbonylalkyl- oder Alkylmercaptocarbonylalkylrest mit jeweils 1 bis 4 Kohlenstoffatomen in einer Alkylgruppe, einen gegebenenfalls durch Halogen, einen Alkyl, Halogenalkyl-, Alkoxy-, Halogenalkoxy-, Alkylmercapto-, Halogenalkyl-mercapto-, Alkylsulfinyl-, Halogenalkylsulfinyl-, Alkylsulfonyl- oder Halogenalkylsulfonylrest mit 1 bis 4 Kohlenstoffatomen, Nitro, Cyano, Azido, Carboxy, Thiocyanato, Hydroxyiminomethyl, Formyl, einen Alkoxycarbonyl- oder Alkylmercaptocarbonylrest mit 2 bis 5 Kohlenstoffatomen, einen N-Alkylcarbamoyl-, N,N-Dialkylcarbamoyl-, N-Alkylcarbamoylamino- oder N,N-Dialkylcarbamoylaminorest mit 1 bis 4 Kohlenstoffatomen in einer Alkylgruppe, einen 4-Alkyl-1,2,4-oxadiazolidin-3,5-dion-2-ylrest, einen Alkanoylamino-, Alkoxycarbonylamino- oder Alkylmercaptocarbonylaminorest mit 2 bis 5 Kohlenstoffatomen, einen N-Alkylsulfamoyl-, N,N-Dialkylsulfamoyl-, N-Alkylsulfamoylamino- oder N,N-Dialkylsulfamoylaminorest mit 1 bis 4 Kohlenstoffatomen in einer Alkylgruppe, einen Alkoxysulfonylrest mit 1 bis 4 Kohlenstoffatomen, einen Alkoxycarbonylaminosulfonylrest mit 2 bis 5 Kohlenstoffatomen oder einen Alkoxycarbonylalkoxy-, Alkylmercaptocarbonylalkoxy-, Alkoxycarbonylalkylmercapto- oder Alkylmercaptocarbonylalkylmercaptorest mit jeweils 1 bis 4 Kohlenstoffatomen in einer Alkylgruppe substituierten Phenylrest, einen gegebenenfalls durch Halogen, Nitro, einen Alkyl-, Halogenalkyl-, Alkoxy-, Halogenalkoxy- oder Halogenalkylmercaptorest mit 1 bis 4 Kohlenstoffatomen substituierten Benzylrest oder einen gegebenenfalls durch Halogen, einen Alkyl-, Alkoxy-, Halogenalkyl-, Halogenalkoxy- oder Halogenalkylmercapto mit 1 bis 4 Kohlenstoffatomen am Phenylring substituierten Phenoxyalkylrest mit 2 bis 4 Kohlenstoffatomen in der Alkylgruppe,

$R^3$ Wasserstoff, einen gesättigten oder ungesättigten, unverzweigten oder verzweigten aliphatischen Rest mit bis zu 10 Kohlenstoffatomen, einen durch Halogen oder Alkoxy mit 1 bis 4 Kohlenstoffatomen substituierten gesättigten, unverzweigten oder verzweigten aliphatischen Rest mit bis zu 10 Kohlenstoffatomen oder einen Cycloalkylrest mit 3 bis 7 Kohlenstoffatomen und

Y Sauerstoff, Schwefel, —SO— oder —$SO_2$— bedeuten, eine starke herbizide Wirkung haben.

In der Formel I stehen $R^1$ und $R^3$ für Wasserstoff, einen gesättigten oder ungesättigten, unverzweigten oder verzweigten aliphatischen Rest, mit bis zu 10 Kohlenstoffatomen, beispielsweise für einen Alkylrest mit bis zu 10 Kohlenstoffatomen, vorzugsweise mit bis zu 4 Kohlenstoffatomen, für einen Alkenyl- oder Alkinylrest mit bis zu 10 Kohlenstoffatomen, vorzugsweise mit bis zu 4 Kohlenstoffatomen, wie Methyl, Ethyl, n-Propyl, Isopropyl, n-Butyl, Isobutyl, sec.-Butyl, tert.-Butyl, n-Pentyl, tert.-Amyl, n-Hexyl, Pentyl-3, 1,2-Dimethyl-n-propyl, 1,3-Dimethyl-n-butyl, 1-Ethyl-2-methyl-n-propyl, 1,2,2-Trimethyl-n-propyl, 1,2-Dimethyl-4-hexyl, Allyl, Methallyl, Crotyl, 2-Ethyl-hex-2-enyl, Hex-5-enyl, 2-Methyl-but-2-enyl, 2-Methyl-but-3-enyl, But-1-en-3-yl, 2-Methyl-but-1-en-4-yl, 2-Methyl-but-2-en-4-yl, 3-Methyl-but-1-en-3-myl, Propargyl, But-1-in-3-yl, But-2-inyl, für einen durch Halogen oder Alkoxy mit 1 bis 4 Kohlenstoffatomen substituierten gesättigten, unverzweigten oder verzweigten aliphatischen Rest mit bis zu 10 Kohlenstoffatomen, vorzugsweise mit bis zu 4-Kohlenstoffatomen, beispielsweise einen Halogenalkylrest mit bis zu 10 Kohlenstoffatomen, vorzugsweise mit 1 bis 4 Kohlenstoffatomen oder einen durch Alkoxy mit 1 bis 4 Kohlenstoffatomen substituierten Alkylrest mit bis zu 10 Kohlenstoffatomen, vorzugsweise mit bis zu 4 Kohlenstoffatomen, wie 2-Chlorethyl, 2-Chlor-n-propyl, 3-Chlor-n-propyl, 2-Chlor-sec.-butyl, 2-Chlor-isobutyl, 2-Fluor-sec.-butyl, 2-Fluor-isobutyl, 2-Fluor-isopropyl, Chlor-tert.-butyl, 2,2,2-Trifluorethyl, 2-Methoxyethyl, 2-Ethoxyethyl, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Methoxy-n-butyl, 4-Methoxy-n-butyl oder für einen Cycloalkylrest mit 3 bis 7 Kohlenstoffatomen, wie Cyclopropyl, Cyclopentyl, Cyclohexyl.

$R^1$ steht außerdem für einen Alkyl- oder Dialkylaminorest mit 1 bis 6 Kohlenstoffatomen, vorzugsweise mit 1 bis 4 Kohlenstoffatomen, in einer Alkylgruppe, wie Methylamino, Dimethylamino, Ethylamino, Isopropylamino, n-Butylamino, Methylethylamino, Diisopropylamino, für einen durch Alkylmercapto mit 1 bis 4 Kohlenstoffatomen substituierten gesättigten, unverzweigten oder verzweigten aliphatischen Rest mit bis zu 10 Kohlenstoffatomen, beispielsweise einen durch Alkylmercapto mit 1 bis 4 Kohlenstoffatomen substituierten, Alkylrest mit bis zu 10 Kohlenstoffatomen, vorzugsweise mit 1 bis 4 Kohlenstoffatomen, wie 2-Methylmercaptoethyl, 2-Ethylmercapto-ethyl, 3-Methylmercapto-n-propyl, 3-Methylmercapto-n-butyl, 1-Methylmercapto-sec.-butyl, Methylmercapto-tert.-butyl, 2-Methylmercapto-n-butyl oder für einen gegebenenfalls durch Halogen, Alkyl oder Alkoxy mit 1 bis 4 Kohlenstoffatomen substituierten Phenylrest oder für einen gegebenenfalls durch Halogen am Phenylring substituierten Benzylrest, wie Phenyl, 4-Chlorphenyl, 3,4-Dichlorphenyl, o-, m-, p-tert.-Butylphenyl, o-, m-, p-Methoxyphenyl, o-, m-, p-Methylphenyl, 4-Methoxy-3-chlorphenyl, 2-Methyl-4-chlorphenyl, Benzyl, 2,6-Dichlorbenzyl, 2-Chlor-6-fluorbenzyl, 2,6-Difluor-benzyl, o-, m-, p-Chlorbenzyl.

$R^2$ in Formel I steht für einen gesättigten oder ungesättigten, unverzweigten oder verzweigten aliphatischen Rest mit bis zu 10 Kohlenstoffatomen, beispielsweise für einen Alkylrest mit bis zu 10 Kohlenstoffatomen, vorzugsweise mit bis zu 4 Kohlenstoffatomen, für einen Alkenyl- oder Alkinylrest mit bis zu 10 Kohlenstoffatomen, vorzugsweise mit bis zu 4 Kohlenstoffatomen, wie Methyl, Ethyl, n-Propyl, Isopropyl, n-butyl, Isobutyl, sec.-Butyl, tert.-Butyl, n-Pentyl, tert.-Amyl, n-Hexyl, Pentyl-3, 1,2-Dimethyl-n-propyl, 1,3-Dimethyl-n-butyl, 1-Ethyl-2-methyl-n-propyl, 1,2,2-Trimethyl-n-propyl, 1,2-Dimethyl-n-hexyl, Allyl, Methallyl, Crotyl, 2-Ethyl-hex-2-enyl, Hex-5-enyl, 2-Methyl-but-2-enyl, 2-Methyl-but-3-enyl, But-1-en-3-yl, 2-Methyl-but-1-en-4-yl, 2-Methyl-but-2-en-4-yl, 3-Methyl-but-1-en-3-yl, Propargyl, But-1-in-3-yl, But-2-inyl, für einen durch halogen, Alkoxy oder Alkylmercapto mit 1 bis 4 Kohlenstoffatomen oder Alkanoyl mit 2 bis 8 Kohlenstoffatomen substituierten, gesättigten oder ungesättigten, unverzweigten oder verzweigten aliphatischen Rest mit bis zu 10 Kohlenstoffatomen, beispielsweise für einen Halogenalkylrest mit bis zu 10 Kohlenstoffatomen, vorzugsweise mit bis zu 4 Kohlenstoffatomen, für einen Halogenalkenyl- oder Halogenalkinylrest mit bis zu 10 Kohlenstoffatomen, vorzugsweise mit bis zu 4 Kohlenstoffatomen, für einen durch Alkoxy oder Alkylmercapto mit 1 bis 4 Kohlenstoffatomen substituierten Alkyl-, Alkenyl- oder Alkinylrest mit bis zu 10 Kohlenstoffatomen, vorzugsweise mit bis zu 4 Kohlenstoffatomen, für einen durch Alkanoyl mit 2 bis 8 Kohlenstoffatomen substituierten Alkyl-, Alkenyl- oder Alkinylrest mit bis zu 10 Kohlenstoffatomen, vorzugsweise mit bis zu 4 Kohlenstoffatomen, wie 2-Chlorethyl, 2-Chlor-n-propyl, 3-Chlor-n-propyl, 2-Chlor-isopropyl, 1-Chlormethyl-n-propyl, 2-Chlor-sec.-butyl, 2-Chlor-isobutyl, 2-Fluor-sec.-butyl, 2-Fluor-isobutyl, 2-Fluor-isopropyl, Chlor-tert.-butyl, 2,2,2-Tribluorethyl, 2-Methoxyethyl, 2-Ethoxyethy, 3-Methoxy-n-propyl, 2-Methoxy-isopropyl, 3-Methoxy-n-butyl, 1-Methoxy-sec.-butyl, Methoxy-tert.-butyl, Ethoxy-tert.-butyl, 2-Methoxy-n-butyl, 4-Methoxy-n-butyl, 3-Chlor-prop-1-enyl, 2-Chlor-prop-1-enyl, 2-Chlor-prop-2-enyl, 3-Chlor-propargyl, 4-Chlor-but-2-inyl, 4-Chlor-but-1-in-3-yl, 3-Methoxy-prop-1-enyl, 2-Methoxy-prop-1-enyl, 2-Methoxy-prop-2-enyl, 3-Methoxypropargyl, 4-Methoxy-but-2-inyl, 4-Methoxy-but-1-in-3-yl, 2-Methylmercapto-ethyl, 2-Ethylmercapto-ethyl, 3-Methylmercapto-n-propyl, 3-Methyl-mercapto-n-butyl, 1-Methylmercapto-sec.-butyl, Methylmercapto-tert.-butyl, 2-Methylmercapto-n-butyl, 3-Methylmercapto-prop-1-enyl, 2-Methylmercapto-prop-1-enyl, Acetylmethyl, Propanoylmethyl, Butanoylmethyl, für einen Cycloalkylrest mit 3 bis 7 Kohlenstoffatomen, wie Cyclopropyl, Cyclopentyl, Cyclohexyl, für einen Alkoxycarbonylalkyl- oder Alkylmercaptocarbonylalkylrest, mit jeweils 1 bis 4 Kohlenstoffatomen in einer Alkylgruppe, wie 1-Ethoxycarbonyl-ethyl, Methylmercaptocarbonylmethyl, für einen gegebenenfalls durch Halogen, einen Alkyl-, Halogenalkyl-, Alkoxy-, Halogenalkoxy-, Alkylmercapto-, Halogenalkylmercapto, Alkylsulfinyl-, Halogenalkylsulfinyl-, Alkylsulfonyl- oder Halogenalkylsulfonylrest mit 1 bis 4 Kohlenstoffatomen, Nitro, Cyano, Azido, Carboxy, Thiocyanato, Hydroxyiminomethyl, Formyl, einen Alkoxycarbonyl- oder Alkylmercaptocarbonylrest mit 2 bis 5 Kohlenstoffatomen, einen N-Alkylcarbamoyl-, N,N-Dialkylcarbamoyl-, N-Alkylcarbamoylamino- oder N,N-Dialkylcarbamoylaminorest mit 1 bis 4 Kohlenstoffatomen in einer Alkylgruppe, einen 4-Alkyl-1,2,4-oxadiazolidin-3,5-dion-2-ylrest, einen Alkanoylamino-, Alkoxycarbonylamino- oder Alkylmercaptocarbonylaminorest mit 2 bis 5 Kohlenstoffatomen, einen N-Alkylsulfamoyl-, N,N-Dialkylsulfamoyl-, N-Alkylsulfamoylamino- oder N,N-Dialkylsulfamoylaminorest mit 1 bis 4 Kohlenstoffatomen in einer Alkylgruppe, einen Alkoxysulfonylrest mit 1 bis 4 Kohlenstoffatomen, einen Alkoxycarbonylaminosulfonyulrest mit 2 bis 5 Kohlenstoffatomen oder einen Alkoxycarbonylalkoxy-, Alkylmercaptocarbonylalkoxy-, Alkoxycarbonylalkylmercapto- oder Alkylmercaptocarbonylalkylmercaptorest mit jeweils 1 bis 4 Kohlenstoffatomen in einer Alkylgruppe,

substituierten Phenylrest, wie o-, m-, p-Trifluormethylphenyl, o-, m-, p-Chlordifluormethoxy, o-, m-, p-Trifluormethoxyphenyl, o-, m-, p-Methylmercaptophenyl, o-, m-, p-Methylsulfinylphenyl, o-, m-, p-Trifluormethylsulfinylphenyl, o-, m-, p-Methylsulfonylphenyl, o-, m-, p-Trifluormethylsulfonylphenyl, o-, m-, p-Nitrophenyl, o-, m-, p-Cyanophenyl, o-, m-, p-Azidophenyl, o-, m-, p-Carboxyphenyl, o-, m-, p-Thiocyanatophenyl, o-, m-, p-N-Hydroxyl-amino-benzyliden, o-, m-, p-Formylphenyl, o-, m-, p-Methoxycarbonylphenyl, o-, m-, p-Methylmercaptocarbonylphenyl, o-, m-, p-(N-Methylcarbamoyl)-phenyl, o-, m-, p-(N,N-Dimethyl-carbamoyl)-phenyl, o-, m-, p-Acetylamino-phenyl, o-, m-, p-Methoxycarbonyl-aminophenyl, o-, m-, p-Methylmercapto-carbonylaminophenyl, o-, m-, p-(N-Methyl-sulfamoyl)-phenyl, o-, m-, p-(N,N-Dimethylsulfamoyl)-phenyl, o-, m-, p-(N-Methyl-sulfamoyl-amino)-phenyl, o-, m-, p-(N,N-Dimethyl-sulfamoyl-amino)-phenyl, o-, m-, p-(N-Methyl-carbamoyl-amino)-phenyl, o-, m-, p-(N,N-Dimethylcarbamoyl-amino)-phenyl, o-, m-, p-Methoxysulfonyl-phenyl, o-, m-, p-Methoxycarbonylaminosulfonylphenyl, o-, m-, p-Methoxycarbonyl-methoxy-phenyl, o-, m-, p-Methoxycarbonyl-methylmercapto-phenyl, o-, m-, p-(1-Methoxycarbonyl-ethoxy)-phenyl, o-, m-, p-(1-Methoxycarbonyl-ethylmercapto)-phenyl, o-, m-, p-Methylmercaptocarbonylmethoxy-phenyl, o-, m-, p-(1-Methylmercaptocarbonyl-ethoxy)-phenyl, o-, m-, p-Brom-phenyl, 2,3-, 2,4-, 2,6-, 3,5-Dichlorphenyl, 2,4,5-Trichlorphenyl, 3,4-Difluorphneyl, 3-Chlor-4-fluorphenyl, 3-Fluor-4-chlorphenyl, 3-Chlor-4-methoxyphenyl, 3-Chlor-4-bromphenyl, 3-Chlor-4-methylphenyl, o-, m-, p-Isopropylphenyl, o-, m-, p-tert.-Butoxyphenyl, 2,4-Dinitrophenyl, 2-Chlor-4-nitrophenyl, 2,4-Dinitro-6-sek.-butylphenyl, 2,6-Dibrom-4-cyanophenyl, 2,6-Dijod-4-cyanophenyl, 2,4-Dinitro-6-methylphenyl, 3-Carboxy-4-nitrophneyl, 2,4-Dinitro-6-tert.-butylphenyl, o-, m-, p-(4'-Methyl-1',2',4'-oxadioazolidin-3',5'-dion-2-yl)-phenyl, 2,4-Dichlor-6-methylphenyl, 2-Chlor-4-trifluormethylphenyl, 3-Methoxycarbo-4-nitrophenyl, o-, m-, p-Trifluormethylmercaptophenyl, o-, m-, p-Chlordifluormethoxyphenyl, o-, m-, p-Chlordifluormethylmercaptophenyl, einen gegebenenfalls durch Halogen, Nitro, einen Alkyl-, Halogenalkyl-, Alkoxy-, Halogenalkoxy- oder Halogenalkylmercaptorest mit 1 bis 4 Kohlenstoffatomen substituierten Benzylrest oder einen gegebenenfalls durch Halogen, einen Alkyl-, Alkoxy-, Halogenalkyl-, Halogenalkoxy- oder Halogenalkylmercapto mit 1 bis 4 Kohlenstoffatomen am Phenylring substituierten Phenoxyalkylrest mit 2 bis 4 Kohlenstoffatomen in der Alkylgruppe, wie 2-Methyl-6-chlorbenzyl, o-, m-, p-Chlorbenzyl, o-, m-, p-Methylbenzyl, o-, m-, p-Methoxybenzyl, o-, m-, p-Nitrobenzyl, o-, m-, p-Trifluormethylbenzyl, o-, m-, p-Trifluormethoxybenzyl, o-, m-, p-chlordifluormethoxybenzyl, o-, m-, p-Trifluormethylmercaptobenzyl, 2-Methyl-6-fluorbenzyl, 3,4-Dichlorbenzyl, 2-(o-, m-, p-Methoxyphenoxy)-ethyl, 2-(o-, m-, p-Chlorphenoxy)-ethyl, 2-(o-, m-, p-Trifluormethylphenoxy)-ethyl, 2-(o-, m-, p-Methylphenoxy)-ethyl.

Bevorzugte Verbindungen der Formel I sind solche, bei denen $R^1$, $R^2$ und $R^3$ Alkyl mit 1 bis 4 Kohlenstoffatomen und Y Sauerstoff oder Schwefel bedeuten, solche, bei denen $R^1$ und $R^3$ Alkyl mit 1 bis 4 Kohlenstoffatomen, $R^2$ einen ungesättigten aliphatischen Rest mit 3 oder 4 Kohlenstoffatomen, beispielsweise einen Alkenylrest mit 3 oder 4 Kohlenstoffatomen, und Y Sauerstoff oder Schwefel bedeuten, solche, bei denen $R^1$ und $R^3$ Alkyl mit 1 bis 4 Kohlenstoffatomen, $R^2$ einen durch Halogen, Nitro, Cyano oder Alkyl mit 1 bis 4 Kohlenstoffatomen substituierten Phenylrest und Y Sauerstoff oder Schwefel bedeuten, oder solche, bei denen $R^1$ und $R^3$ Alkyl mit 1 bis 4 Kohlenstoffatomen, $R^2$ einen gegebenenfalls durch Halogen, einen Alkyl-, Alkoxy- oder Halogenalkoxyrest mit 1 bis 4 Kohlenstoffatomen substituierten Benzylrest und Y Sauerstoff oder Schwefel bedeuten.

Man erhält die 2H-1,2,4,6-Thiatriazin-1,1-dioxide der Formel I durch Umsetzung von Verbindungen der Formel

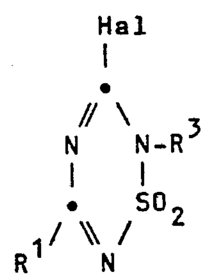

(II)

in der $R^1$ und $R^3$ die obengenannten Bedeutungen haben und Hal Halogen bedeutet, mit einer Verbindung der Formel

$$H—Y—R^2 \qquad (III),$$

in der $R^2$ und Y die obengenannten Bedeutungen haben oder einem Alkali-, Erdalkali- oder Ammoniumsalz, einer Verbindung der Formel III, gegebenenfalls in einem inerten organischen Lösungsmittel und gegebenenfalls in Anwesenheit eines Säureakzeptors bei einer Temperatur zwischen − 50 und + 150 °C. Die Umsetzung kann drucklos oder unter Druck kontinuierlich oder diskontinuierlich durchgeführt werden.

Verwendet man 3-Chlor-5-methyl-2-isopropyl-2H-1,2,4,6-thiatriazin-1,1-dioxid und Propanol als Ausgangsmaterialien, so kann der Reaktionsablauf durch folgendes Formelschema wiedergegeben werden :

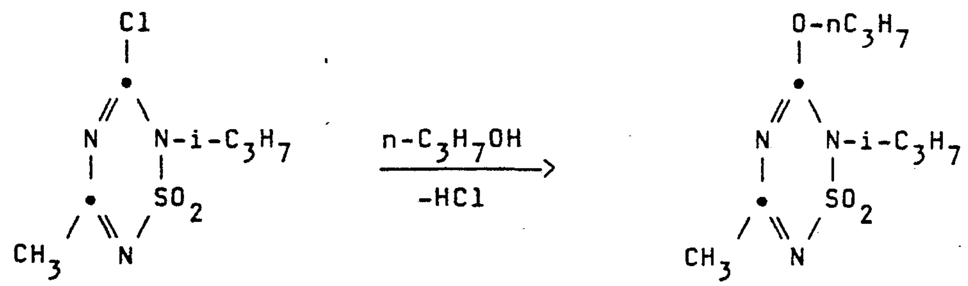


Verwendet man 3-Chlor-5-ethyl-2-isopropyl-2H-1,2,4,6-thiatriazin-1,1-dioxid und 4-Chlorbenzolsulfinsäure-natriumsalz, so kann der Reaktionsablauf durch folgendes Formelschema wiedergegeben werden :

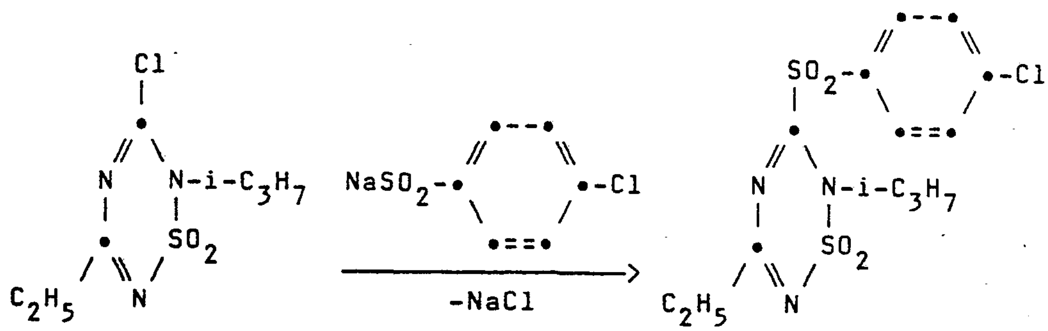


Verwendet man 3-Chlor-5-methyl-2-isopropyl-2H-1,2,4,6-thiatriazin-1,1-dioxid und 2,4-Dichlorphenol, so kann der Reaktionsablauf durch folgendes Formelschema wiedergegeben werden :

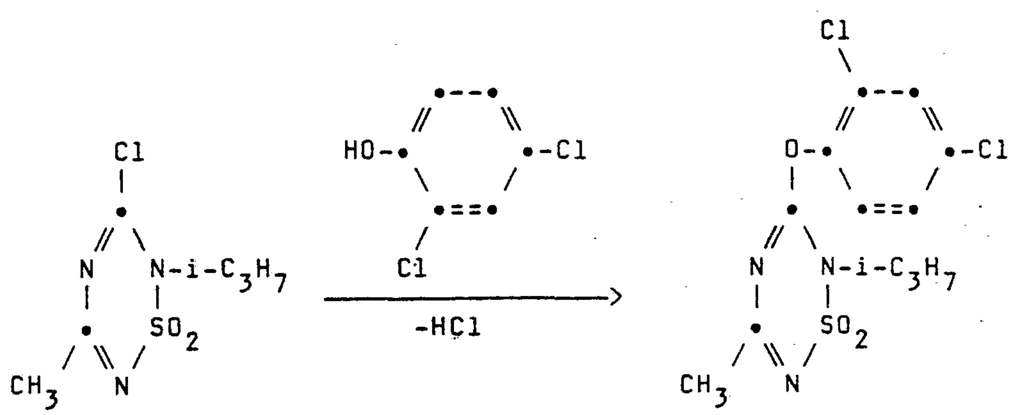


Zweckmäßigerweise verwendet man für die Umsetzung unter den jeweiligen Reaktionsbedingungen inerte Lösungs- oder Verdünnungsmittel. Als Lösungsmittel kommen beispielsweise Halogenkohlenwasserstoffe, insbesondere Chlorkohlenwasserstoffe, z. B. Tetrachlorethylen, 1,1,2,2,- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol ; Ether, z. B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether ; Nitrokohlenwasserstoffe wie Nitromethan, Nitroethan, Nitrobenzol, o-, m-, p-Chlornitrobenzol, o-Nitrotoluol ; Nitrile wie Acetonitril, Butyronitril, Isobutyronitril, Benzonitril, m-Chlorbenzonitril ; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2, 3,3-Trimethylpentan, Octan ; Ester, z. B. Ethylacetat, Acetessigester, Isobutylacetat ; Amide, z. B. Formamid, Methylformamid, Dimethylformamid ; Ketone, z. B. Aceton, Methylethylketon, gegebenenfalls auch Wasser und entsprechende Gemische in Betracht. Zweckmäßig verwendet man das Lösungsmittel in einer Menge von 100 bis 2 000 Gew.%, vorzugsweise von 200 bis 700 Gew.%, bezogen auf Ausgangsstoff II.

Als Säureakzeptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z. B. als basische Verbindungen in Frage : Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethyleninmin, Pyridin, Chinolin, α-Picolin, β-Picolin, γ-Picolin, Isochinolin, Pyrimidin, Acridin, N,N, N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßigerweise verwendet man den Säureakzeptor in einem Über- oder Unterschuß von bis zu 20 %, bezogen auf den Ausgangsstoff II. Man kann jedoch auch den entstehenden Halogenwasserstoff durch Eingasen eines Inertgases, beispielsweise von Stickstoff, entfernen.

Die zur Umsetzung benötigten Ausgangsstoffe III werden im allgemeinen in einem Über- oder Unterschuß von bis zu 20 %, bezogen auf den Ausgangsstoff II, eingesetzt. Man kann den Ausgangsstoff III jedoch auch direkt als Lösungsmittel verwenden. Man kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Zweckmäßigerweise wird das Verfahren zur Herstellung der neuen Verbindungen so durchgeführt, daß man den Ausgangsstoff II, gegebenenfalls in einem der vorgenannten Verdünnungsmittel vorlegt und dann den Ausgangsstoff III und einen Säureakzeptor gleichzeitig oder nacheinander zugibt. Man kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 10 Stunden bei − 50 bis 150 °C, vorzugsweise 0 bis 120 °C, insbesondere 10 bis 50 °C, nach.

Verwendet man ein Inertgas zur Entfernung des Halogenwasserstoffes, so rührt man zweckmäßigerweise, 0,2 bis 10 Stunden bei 40 bis 100 °C nach.

Aus dem Reaktionsgemisch wird der Endstoff I in üblicher Weise, z. B. nach Abdestillieren von Lösungsmittel oder überschüssigem Ausgangsstoff III oder direkt durch Absaugen, isoliert. Der verbleibende Rückstand wird in diesem Fall zur Entfernung saurer Verunreinigungen mit Wasser bzw. verdünntem Alkali gewaschen und getrocknet. Im Falle von mit Wasser nicht mischbaren Verdünnungsmitteln kann man auch direkt das Reaktionsgemisch mit Wasser bzw. mit verdünntem Alkali extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation gereinigt werden.

Man erhält die Ausgangsverbindungen der Formel II durch Umsetzung von Verbindungen der Formel

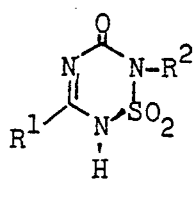

(IV)

in der $R^1$ und $R^2$ die im Anspruch 1 genannten Bedeutungen haben, oder deren Alkalimetallsalzen oder Erdalkalimetallsalzen mit einem Säurehalogenid der Phosphorsäure, phosphorigen Säure, Kohlensäure,

Oxalsäure oder schwefligen Säure gegebenenfalls in Gegenwart eines Lösungs- oder Verdünnungsmittels und gegebenenfalls in Gegenwart eines Reaktionsbeschleunigers.

Zweckmäßigerweise verwendet man für die Umsetzung unter den Reaktionsbedingungen inerte Lösungs- oder Verdünnungsmittel, wie Halogenkohlenwasserstoffe, insbesondere Chlorkohlwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Tetrachlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, o-, m-, p-Dichlorbenzol, o-, m-, p-Dibrombenzol, o-, m-, p-Chlortoluol, 1,2,4-trichlorbenzol, oder ether, z. B. Ethylpropylether, Methyl-tert.-butylether, n-Butylether, Diisopropylether, Anisol, Dioxan, Ethylenglykoldimethyl-ether, oder nitro-kohlenwasserstoffe, z. B. Nitrobenzol, o-, m-, p-Chlornitrobenzol, o-Nitrotoluol, oder Nitrile, z. B. Acetonitril, Butyronitril, Isobutyronitril, Benzonitril, m-Chlorbenzonitril, oder aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, oder Ester, z. B. Ethylacetat, und entsprechende Gemische. Geeignete Lösungsmittel sind ferner anorganische Säurechloride, z. B. Phosphoroxychlorid, oder entsprechende Mischungen mit inerten Chlorkohlenwasserstoffen, wie 1,2-Dichlorethan. Zweckwäßigerweise verwendet man das Lösungsmittel in einer Menge von 100 bis 2 000 Gew.-%, vorzugsweise von 200 bis 700 Gew.-%, bezogen auf den Ausgangsstoff der Formel IV.

Bevorzugte Säurehalogenide sind Thionylchlorid, Schwefeltetrafluorid, Phosgen, Oxalsäurechlorid, Phosphortribromid und insbesondere Phosphorpentachlorid, Phosphortrichlorid und Phosphoroxichlorid. Die Umsetzung wird im allgemeinen mit einer Menge von 1,0 bis 1,5, vorzugsweise 1,05 bis 1,2 Mol, Säurehalogenid, bezogen auf Ausgangsstoff IV, durchgeführt ; im Falle der Verwendung fünfwertiger Phosphorhalogenverbindungen verwendet man 0,7 bis 1,5, vorzugsweise 1,0 bis 1,2 Mol, Phosphorpentahalogenid, bezogen auf Ausgangsstoff IV.

Im Falle der Verwendung eines Phosphor(V) halogenids als Halogenierungsagens empfiehlt sich die Verwendung eines Phosphoroxihalogenids als Verdünnungsmittel, das zweckmäßigerweise in einer Menge von 1 bis 10 Mol Phosphoroxihalogenid, bezogen auf Ausgangsstoff IV, eingesetzt wird.

Hierbei kann das Phosphor(V)-halogenid auch direkt in situ hergestellt werden, indem man beispielsweise eine Mischung eines Phosphor(III)-halogenids in Phosphoroxyhalogenid oder einem der obengenanten inerten Lösungsmittel mit der erforderlichen stöchiometrischen Menge aktivem Halogen umsetzt, beispielsweise nach dem in der US-PS 1 906 440 beschriebenen Verfahren, und dann nach Zugabe des Ausgangsstoffes IV die Umsetzung durchführt.

Als Reaktionsbeschleuniger kann man vorteilhafterweise ein am Stickstoffatom disubstituiertes, gegebenenfalls cyclisches Carbonsäureamid, einen tetraalkylsubstituierten Harnstoff, ein tertiäres Amin, in einer Menge von 1 bis 10 Gew.%, bezogen auf den Ausgangsstoff IV, verwenden. Auch Gemische der genannten Katalysatoren kommen für die Reaktion in Betracht. Ferner kann man auch Salze von Diaminen, z. B. die Hydrochloride der Amine, oder ein quaternäres Salz eines Amins verwenden. Bevorzugte Katalysatoren sind :

Triethylamin, Pyridin, N,N-Dimethylanilin, N-Ethyl-piperidin, N-Methylpyrrolidin, α,β- oder γ-Picolin, Chinolin, Isochinolin, Chinazolin, Chinoxalin, N-Propyldiisopropylamin, 2,6- und 2,4-Lutidin, N-(4-Pyridyl)-pyridiniumchlorid-hydrochlorid, p-Dimethylaminopyridin, Pyrimidin, Acridin, Dimethylformamid, Diethylformamid, Ameisensäure-N-methylanilid, N,N-Dimethylacetamid, N-Methylpyrrolidon, Tetramethylharnstoff.

Die als Ausgangsstoffe der Formel IV benötigten 2H-1,2,4,6-Thiatriazin(3)on-1,1-dioxide sind teilweise bekannt oder lassen sich durch Umsetzung von N-Carboalkoxy-amidinen mit Aminosulfonylhalogeniden herstellen.

## Beispiel 1

2-Ethyl-5-methyl-3-methoxy-2H-1,2,4,6-thiatriazin-1,1-dioxid (Nr. 1)

225 Teile 2-Ethyl-5-methyl-2H-1,2,4,6-thiatriazin(3)on-1,1-dioxid wurden innerhalb 2 Minuten unter Rühren in eine Mischung von 1 300 Teilen Phosphoroxichlorid und 292 Teilen Phosphorpentachlorid gegeben. Das Reaktionsgemisch wurde 7 Stunden unter Rückfluß gerührt und dann unter vermindertem Druck eingeengt. Das verbleibende Öl wurde in 350 Teilen 1,2-Dichlorethan aufgenommen und über neutrales Aluminiumoxid (Aktivität I) chromatographiert. Nach dem Einengen wurden 230 Teile (Ausbeute : 93 % d.Th.) 3-Chlor-2-ethyl-5-methyl-2H-1,2,4,6-thiatriazin-1,1-dioxid vom Fp. 57 bis 59 °C erhalten. (NMR (60 MHz, $CDCl_3$)

$CH_3$-C 2,3 δ, N-$CH_2$ 3,68-4,03 δ (q),

$CH_3$-C 1,2-1,43 δ (t).

19,8 Teile 30 prozentiges Natriummethylat werden unter Rühren bei 20 bis 25 °C innerhalb von 10 Minuten zu einer Lösung von 20,95 Teilen 3-Chlor-2-ethyl-5-methyl-2H-1,2,4,6-thiatriazin-1,1-dioxid in 120 Teilen THF gegeben. Nach drei Stunden Rühren bei 25 °C wird das Reaktionsgemisch eingeengt, in Essigester aufgenommen und zweimal mit verdünnter Natriumcarbonatlösung extrahiert. Die organische

Phase wird abgetrennt, getrocknet und über Kieselgel chromatographiert. Nach dem Einengen unter vermindertem Druck werden 14,5 Teile 2-Ethyl-5-methyl-3-methoxy-2H-1,2,4,6-thiatriazin-1,1-dioxid vom Fp. 50 bis 53 °C erhalten.

## Beispiel 2

2,5-Dimethyl-3-(4'-nitrophenoxy)-2H-1,2,4,6-thiatriazin-1,1-dioxid (Nr. 2)

212 Teile 2,5-Dimethyl-2H-1,2,4,6-thiatriazin(3)on-1,1-dioxid wurden bei Raumtemperatur in eine Mischung aus 292 Teilen Phosphorpentachlorid und 1 400 Teilen Phosphoroxichlorid unter Rühren eingeführt und innerhalb 30 Minuten auf 110 °C erhitzt. Nach 6 Stunden Rühren unter Rückfluß wurde das Reaktionsgemisch unter vermindertem Druck eingeengt, wobei 234 Teile (Ausbeute : 100 % d. Th.) 3-Chlor-2,5-dimethyl-2H-1,2,4,6-thiatriazin-1,1-dioxid vom Fp. 86 bis 90 °C erhalten wurden (NMR (60 MHz, $CDCl_3$) : $CH_3$-C 2,4 δ, $CH_3$-N 3,68 δ).

12,8 Teile p-Nitrophenol in 30 Teilen Aceton und 9,3 Teilen Triethylamin werden über zwei Zuführungen innerhalb von 15 Minuten unter Rühren bei 20 bis 25 °C zu einer Lösung von 18 Teilen 3-Chlor-2,5-dimethyl-2H-1,2,4,6-thiatriazin-1,1-dioxid in 150 Teilen Aceton gegeben. Nach einer Stunde Rühren bei Raumtemperatur wird vom ausgefallenen Niederschlag abgesaugt und das Filtrat unter vermindertem Druck eingeengt. Der Rückstand wird in Methylenchlorid aufgenommen, zweimal mit 0,5 n Natronlauge extrahiert und über Magnesiumsulfat getrocknet. Nach dem Chromatographieren über neutralem Aluminiumoxid und Einengen unter vermindertem Druck werden 18,5 Teile 2,5-Dimethyl-3-p-nitrophenoxy-2H-1,2,4,6-thiatriazin-1,1-dioxid vom Fp. 194 bis 197 °C erhalten.

## Beispiel 3

2,5-Dimethyl-3-m-methoxycarbamoyl-phenoxy-2H-1,2,4,6-thiatriazin-1,1-dioxid (Nr. 3)

18,4 Teile m-Hydroxyphenyl-N-methylcarbamat und 11,1 Teile Triethylamin werden unter Rühren bei 25 bis 30 °C innerhalb 10 Minuten zu einer Lösung von 19,5 Teilen 3-Chlor-2,5-dimethyl-2H-1,2,4,6-thiatriazin-1,1-dioxid in 140 Teilen Essigester gegeben. Nach 5 Stunden Rühren bei Raumtemperatur wird vom ausgefallenen Niederschlag abgesaugt, und das Filtrat wird mit verdünnter Natriumcarbonatlösung extrahiert. Aus der organischen Phase werden nach dem Trocknen und Einengen 21,8 Teile farbloses 2,5-Dimethyl-3-m-methoxycarbamoyl-phenoxy-2H-1,2,4,6-thiatriazin-1,1-dioxid vom Fp. 181 bis 184 °C erhalten.

## Beispiel 4

2,5-Dimethyl-3-[p-(1-methoxycarbonyl-ethoxy)-phenoxy]-2H-1,2,4,6-thiatriazin-1,1-dioxid (Nr. 205)

19,6 Teile 3-Chlor-2,5-dimethyl-2H-1,2,4,6-thiatriazin-1,1-dioxid in 60 Teilen Essigester, 19,6 Teile α-(p-Hydroxyphenoxy)-propionsäuremethylester in 60 Teilen Essigester und 7,9 Teile Pyridin werden getrennt unter Rühren bei 0 bis 10 °C innerhalb 10 Minuten zu 140 Teilen Essigester zugegeben. Nach einer Stunde Rühren bei Raumtemperatur wird vom ausgefallenen Niederschlag abgesaugt, einmal mit 0,5 n Salzsäure und zweimal mit verdünnter Natriumcarbonatlösung extrahiert. Nach dem Trocknen, Chromatographieren über neutralem Aluminiumoxid und Einengen unter vermindertem Druck werden 21 Teile 2,5-Dimethyl-3-[p-(1-methoxycarbonyl-ethoxy)-phenoxy]-2H-1,2,4,6-thiatriazin-1,1-dioxid vom Fp. 106 bis 109 °C erhalten.

In entsprechender Weise wurden die folgenden 2H-1,2,4,6-Thiatriazin-1,1-dioxide der Formel I erhalten :

(Siehe Tabelle Seite 9 ff.)

| Verbindung Nr. | $R^1$ | Y | $R^2$ | $R^3$ | Fp[°C]/$n_D^{25}$ |
|---|---|---|---|---|---|
| 4 | $CH_3$ | O | $CH_3$ | H | |
| 5 | $CH_3$ | O | $CH_3$ | $CH_3$ | 97-100 |
| 6 | $CH_3$ | O | $C_2H_5$ | $CH_3$ | 1.4990 |
| 7 | $CH_3$ | O | $C_2H_5$ | $C_2H_5$ | |
| 8 | $CH_3$ | O | $C_2H_5$ | i-$C_3H_7$ | |
| 9 | $CH_3$ | O | $CH_2$-CH=$CH_2$ | $C_2H_5$ | |
| 10 | $CH_3$ | S | $CH_3$ | $CH_3$ | |
| 11 | $CH_3$ | S | $CH_2$-CH=$CH_2$ | $CH_3$ | |
| 12 | $CH_3$ | $SO_2$ | $CH_3$ | $CH_3$ | |
| 13 | $CH_3$ | O | $CH_2$-CH=$CH_2$ | H | |
| 14 | $C_2H_5$ | O | $CH_3$ | $CH_3$ | 83- 84 |
| 15 | $C_2H_5$ | O | $C_2H_5$ | $CH_3$ | |
| 16 | $C_2H_5$ | O | $C_2H_5$ | $C_2H_5$ | |
| 17 | $C_2H_5$ | O | $CH_2$-CH=$CH_2$ | $C_2H_5$ | |
| 18 | $C_2H_5$ | O | 1-Propin-yl-3 | $CH_3$ | 78- 80 |
| 19 | $C_2H_5$ | O | $CH_2$-CH=$CH_2$ | $CH_3$ | 1.5018 |
| 20 | $C_2H_5$ | O | 4-Methylbenzyl | $CH_3$ | 87-91 |
| 21 | $C_2H_5$ | O | CH=CH-$CH_2$Cl | $CH_3$ | |
| 22 | $C_2H_5$ | O | CH=CCl-$CH_3$ | $CH_3$ | |
| 23 | n-$C_3H_7$ | O | $CH_2$-$CH_2$-O-$CH_3$ | $CH_3$ | |
| 24 | n-$C_3H_7$ | O | 1-Chlor-2-butin-yl-4 | $CH_3$ | |

| Verbindung Nr. | $R^1$ | Y | $R^2$ | $R^3$ | Fp[°C]/$n_D^{25}$ |
|---|---|---|---|---|---|
| 25 | $i-C_3H_7$ | O | 1-Methoxy-2-butin-yl-4 | $C_2H_5$ | |
| 26 | $CH_3$ | O | 1-Methylmercapto-but-2-in-4-yl | $CH_3$ | |
| 27 | $n-C_3H_7$ | O | 1-Chlor-but-3-in-2-yl | $C_2H_5$ | |
| 28 | $CH_3$ | S | $CH_2-CCl=CH_2$ | $CH_3$ | |
| 29 | $C_2H_5$ | O | $CH_2-CH=CH-CO-CH_3$ | $CH_3$ | |
| 30 | $CH_3$ | O | $CH_2-CO-CH_3$ | $CH_3$ | 1.5092 |
| 31 | $CH_3$ | O | $CH_2-CH_2-S-CH_3$ | $CH_3$ | |
| 32 | $i-C_3H_7$ | O | $CH_3$ | $CH_3$ | |
| 33 | $n-C_4H_9$ | O | $C_2H_5$ | $CH_3$ | |
| 34 | $sek-C_4H_9$ | O | $n-C_3H_7$ | $CH_3$ | |
| 35 | $CH_3$ | O | 2-(3-Chlorphenoxy)-ethyl | $CH_3$ | 112-116 |
| 36 | $CH_3$ | O | $CH(CH_3)CO_2C_2H_5$ | $CH_3$ | 70- 73 |
| 37 | $CH_3$ | O | 4-Trifluormethylphenyl | $CH_3$ | |
| 38 | $CH_3$ | O | 3-Difluormethoxyphenyl | $CH_3$ | |
| 39 | $CH_3$ | S | 3-Chlorphenyl | $CH_3$ | |
| 40 | $CH_3$ | O | $n-C_3H_7$ | $CH_3$ | |
| 41 | $C_2H_5$ | O | $n-C_4H_9$ | $C_2H_5$ | |
| 42 | $CH_3$ | O | 4-Methylmercaptophenyl | $C_2H_5$ | |
| 43 | $CH_3$ | O | 4-Methoxyphenyl | $CH_3$ | |
| 44 | $CH_3$ | O | $CH_3$ | $C_2H_5$ | |

| Verbindung Nr. | $R^1$ | Y | $R^2$ | $R^3$ | Fp[°C]/$n_D^{25}$ |
|---|---|---|---|---|---|
| 45 | $i-C_3H_7$ | O | Prop-2-inyl | $CH_3$ | 1.5060 |
| 46 | $n-C_4H_9$ | O | $C_2H_5$ | $C_2H_5$ | |
| 47 | $CH_3$ | O | $CH_2-CH=CH-CH_3$ | $CH_3$ | 1.4891 |
| 48 | $CH_3$ | O | 2-Butin-yl-4 | $CH_3$ | |
| 49 | $CH_3$ | O | But-1-in-3-yl | $CH_3$ | |
| 50 | $C_2H_5$ | O | But-1-in-3-yl | $CH_3$ | |
| 51 | $CH_3$ | O | $CH_2-CH=CH_2$ | $CH_3$ | 1.5124 |
| 52 | $i-C_3H_7$ | O | $CH_2-CO-CH_3$ | $CH_3$ | |
| 53 | $CH_2-CH=CH_2$ | O | $CH_3$ | $CH_3$ | |
| 54 | Propargyl | O | $CH_3$ | $C_2H_5$ | |
| 55 | $CH_2-CH_2-Cl$ | O | $C_2H_5$ | $CH_3$ | |
| 56 | Cyclohexyl | O | $CH_3$ | $i-C_3H_7$ | |
| 57 | 3-Chlorphenyl | O | $CH_3$ | $CH_3$ | |
| 58 | $CH_3$ | O | $i-C_3H_7$ | $CH_3$ | 1.4819 |
| 59 | $C_2H_5$ | S | $n-C_4H_9$ | $C_2H_5$ | |
| 60 | $n-C_3H_7$ | O | $C_2H_5$ | $CH_3$ | |
| 61 | $CH_3$ | O | 4-Methylphenyl | $C_2H_5$ | |
| 62 | $CH_3$ | O | 4-Trifluormethylmercapto phenyl | $CH_3$ | |
| 63 | $CH_3$ | O | 3-Methoxybenzyl | $CH_3$ | 82- 85 |
| 64 | $C_2H_5$ | O | $C_2H_5$ | $i-C_3H_7$ | |

| Verbindung Nr. | $R^1$ | Y | $R^2$ | $R^3$ | Fp[°C]/$n_D^{25}$ |
|---|---|---|---|---|---|
| 65 | tert-$C_4H_9$ | O | $CH_3$ | $CH_3$ | |
| 66 | $C_2H_5$ | O | $CH_3$ | i-$C_3H_7$ | |
| 67 | $C_2H_5$ | $SO_2$ | $CH_3$ | $CH_3$ | |
| 68 | $CH_3-O-CH_2CH_2$ | O | $CH_3$ | $CH_3$ | |
| 69 | $C_2H_5$ | O | $C_2H_5$ | $CH_3$ | |
| 70 | $C_6H_5-CH_2$ | O | $CH_3$ | $CH_3$ | |
| 71 | 4-Chlorbenzyl | O | $CH_3$ | $CH_3$ | |
| 72 | 4-Methoxy-phenyl | O | $C_2H_5$ | $CH_3$ | |
| 73 | i-$C_3H_7$ | O | $CH_3$ | n-$C_3H_7$ | |
| 74 | $CH_3$ | S | Prop-1-in-3-yl | $CH_3$ | |
| 75 | $CH_3$ | O | $CH_3$ | i-$C_3H_7$ | |
| 76 | $CH_3$ | O | $CH_3$ | sek.-$C_4H_9$ | |
| 77 | $CH_3$ | O | $C_2H_5$ | tert.-$C_4H_9$ | |
| 78 | $CH_3$ | O | 1-Propin-yl-3 | H | |
| 79 | $C_2H_5$ | O | $CH_2-CH=CH-\overset{\overset{O}{\|}}{C}-CH_3$ | $C_2H_5$ | |
| 80 | $CH_3-S-CH_2-CH_2$ | O | $CH_3$ | $CH_3$ | |
| 81 | $CH_3$ | O | $CH_2-CH=CH_2$ | $CH_2-CH=CH_2$ | |
| 82 | $CH_3$ | O | 4-Methylbenzyl | $CH_3$ | 82- 84 |
| 83 | $CH_3$ | O | $CH_3$ | Cyclohexyl | |

| Verbindung Nr. | $R^1$ | Y | $R^2$ | $R^3$ | Fp[°C]/$n_D^{25}$ |
|---|---|---|---|---|---|
| 84 | $C_2H_5$ | O | 3-Methoxybenzyl | $CH_3$ | 82- 84 |
| 85 | $CH_3$ | O | $CH_3$ | $CH_2-O-CH_3$ | |
| 86 | $CH_3$ | O | $CH_3$ | $CH_2-CH_2-Cl$ | |
| 87 | $C_2H_5$ | O | $CH_3$ | $CH_2-CH_2-F$ | |
| 88 | $CH_3$ | O | 4-Nitrophenyl | $CH_3$ | |
| 89 | $CH_3$ | O | 4-Methylsulfinyl-phenyl | $CH_3$ | |
| 90 | $CH_3$ | O | Prop-1-in-3-yl | $CH_3$ | 56- 60 |
| 91 | $CH_3$ | O | 4-Cyanophenyl | $CH_3$ | |
| 92 | $CH_3$ | O | 4-Thiocyanatophenyl | $CH_3$ | |
| 93 | i-$C_3H_7$ | O | 3-Carboxyphenyl | $CH_3$ | |
| 94 | $CH_3$ | O | 4-Nitro-3-carboxyphenyl | $CH_3$ | |
| 95 | $CH_3$ | O | 2-Methyl-4,6-dinitrophenyl | $CH_3$ | |
| 96 | $C_2H_5$ | S | 4-Nitrophenyl | $CH_3$ | |
| 97 | n-$C_3H_7$ | O | 4-Methylsulfonylphenyl | $CH_3$ | |
| 98 | i-$C_4H_9$ | O | Prop-1-in-3-yl | $CH_3$ | 1.5030 |
| 99 | $CH_3$ | O | 3,5-Dichlorphenyl | $C_2H_5$ | |
| 100 | $CH_3$ | O | 2,5-Dichlorphenyl | n-$C_3H_7$ | |
| 101 | $CH_3$ | S | 4-Methoxycarbonylphenyl | $CH_3$ | |
| 102 | $CH_3$ | O | 2,4,6-Trichlorphenyl | $CH_3$ | |
| 103 | $CH_3$ | O | 4-Methylmercapto-carbonyl-phenyl | $C_2H_5$ | |

| Verbindung Nr. | $R^1$ | Y | $R^2$ | $R^3$ | Fp[°C]/$n_D^{25}$ |
|---|---|---|---|---|---|
| 104 | $C_2H_5$ | O | 4-(N-Methylcarbamoyl)-phenyl | $C_2H_5$ | |
| 105 | $CH_3$ | O | 4-(N,N-Dimethylcarbamoyl)--phenyl | $CH_3$ | |
| 106 | $CH_3$ | O | 2,4-Dinitro-6-tert.-butyl-phenyl | $CH_3$ | |
| 107 | $CH_2=CH-CH_2$ | O | 3-Methoxycarbonylaminophenyl | $C_2H_5$ | |
| 108 | i-$C_3H_7$ | O | 4-Methoxycarbonylaminophenyl | $CH_3$ | |
| 109 | $CH_3$ | O | 2,4-Dinitro-6-sek.-butyl-phenyl | $CH_3$ | 128-133 |
| 110 | n-$C_3H_7$ | O | 4-Methylmercaptocarbonyl-aminophenyl | $CH_3$ | |
| 111 | $CH_3$ | O | 4-(N-Methylsulfamoylamino)--phenyl | $CH_3$ | |
| 112 | $CH_3$ | O | 2,6-Dibrom-4-cyanophenyl | $CH_3$ | 179-182 |
| 113 | $C_2H_5$ | O | 4-(N,N-Dimethylsulfamoyl-amino)-phenyl | $C_2H_5$ | |
| 114 | $CH_3$ | O | 4-Formylphenyl | $C_2H_5$ | |
| 115 | $CH_3$ | O | 4-Trifluormethylbenzyl | $CH_3$ | 115-117 |
| 116 | $CH_3$ | O | 2,6-Dijod-4-cyanophenyl | $CH_3$ | |
| 117 | $CH_3$ | O | 2-Nitrophenyl | $C_2H_5$ | |
| 118 | $CH_3$ | O | 3-Nitrophenyl | $CH_3$ | |
| 119 | $C_2H_5$ | O | 4-(N-Hydroxyiminomethyl)--phenyl | $CH_3$ | |

| Verbindung Nr. | $R^1$ | Y | $R^2$ | $R^3$ | Fp[°C]/$n_D^{25}$ |
|---|---|---|---|---|---|
| 120 | $CH_3$ | O | 3-Methylsulfonyl-phenyl | $CH_3$ | |
| 121 | $C_2H_5$ | O | 4-(N,N-Dimethylsulfamoyl-phenyl | $CH_3$ | |
| 122 | $CH_3$ | O | 4-Chlorphenyl | $CH_3$ | |
| 123 | $CH_3$ | | 3-Carboxymethoxyphenyl | i-$C_3H_7$ | |
| 124 | $C_2H_5$ | O | 3-Methoxycarbonyl-methoxy-phenyl | $CH_3$ | |
| 125 | $CH_3$ | O | 4-Nitrophenyl | $C_2H_5$ | 196-198 |
| 126 | $CH_3$ | O | 4-Methoxycarbonylmethyl-mercapto-phenyl | $CH_3$ | |
| 127 | $C_2H_5$ | O | 3-Methylmercaptocarbonyl-methylmercapto-phenyl | $CH_3$ | |
| 128 | $CH_3$ | O | 4-tert.-Butylphenyl | $CH_3$ | |
| 129 | $C_2H_5$ | S | 2,6-Dimethylphenyl | $CH_3$ | |
| 130 | $CH_3$ | O | 4-Chlorbenzyl | $CH_3$ | 86- 90 |
| 131 | $CH_3$ | O | 4-Cyanophenyl | $CH_3$ | |
| 132 | $CH_3$ | O | 3,4-Difluorphenyl | $CH_3$ | |
| 133 | $CH_3$ | O | 3-tert.-Butylphenyl | $CH_3$ | |
| 134 | $C_2H_5$ | O | 4-tert.-Butylphenyl | $CH_3$ | |
| 135 | $CH_3$ | O | 2,4-Dichlor-6-methylphenyl | $CH_3$ | |
| 136 | $C_2H_5$ | O | 2-Chlor-4-nitrophenyl | $CH_3$ | |
| 137 | $CH_3$ | O | 4-Nitrophenyl | H | |

| Verbindung Nr. | $R^1$ | Y | $R^2$ | $R^3$ | Fp[°C]/$n_D^{25}$ |
|---|---|---|---|---|---|
| 138 | $CH_3$ | O | 4-Trifluormethyl-2-chlor-phenyl | $CH_3$ | |
| 139 | $CH_3$ | O | 3-Chlor-4-methoxyphenyl | H | |
| 140 | i-$C_4H_9$ | O | $CH_3$ | $CH_3$ | 1.4935 |
| 141 | $CH_3$ | O | 3-Methoxycarbonylamino-phenyl | $CH_3$ | |
| 142 | $CH_3$ | O | 3-Methylaminocarbonylamino-phenyl | $CH_3$ | |
| 143 | $C_2H_5$ | O | 4-(1-Methoxycarbonyl)-ethoxy-phenyl | $CH_3$ | |
| 144 | $CH_3$ | O | 3,4-Dichlorbenzyl | $CH_3$ | 148-150 |
| 145 | $C_2H_5$ | O | 4-Nitrophenyl | $CH_3$ | 146-148 |
| 146 | i-$C_3H_7$ | O | 2-Chlor-4-nitrophenyl | $CH_3$ | |
| 147 | $C_2H_5$ | O | 4-Nitrophenyl | i-$C_3H_7$ | |
| 148 | $CH_3$ | O | 4-Nitro-3-methoxycarbonyl-phenyl | $CH_3$ | |
| 149 | sek.-$C_4H_9$ | O | 4-Nitrophenyl | i-$C_3H_7$ | |
| 150 | $C_2H_5$ | S | Benzyl | $CH_3$ | |
| 151 | $CH_3$ | O | 4-Nitrophenyl | i-$C_3H_7$ | |
| 152 | $CH_3$ | O | 4-Nitrophenyl | n-$C_3H_7$ | |
| 153 | i-$C_3H_7$ | O | $CH_3$ | $CH_3$ | 1.4942 |
| 154 | $CH_3$ | O | Benzyl | $CH_3$ | 121-124 |
| 155 | $CH_3$ | O | 3-Trifluormethoxy-phenyl | $CH_3$ | |

| Verbindung Nr. | $R^1$ | Y | $R^2$ | $R^3$ | Fp[°C]/$n_D^{25}$ |
|---|---|---|---|---|---|
| 156 | $CH_3$ | O | sek.-Butyl | $CH_3$ | |
| 157 | $C_2H_5$ | S | 1-Propin-yl-3 | $CH_3$ | |
| 158 | $CH_3$ | O | 1-Propin-yl-3 | $C_2H_5$ | 1.5121 |
| 159 | $C_2H_5$ | O | 1-Propin-yl-3 | $C_2H_5$ | |
| 160 | $C_2H_5$ | O | Benzyl | $CH_3$ | 88-90 |
| 161 | $CH_3$ | O | 2,6-Dichlorbenzyl | $CH_3$ | 127-130 |
| 162 | n-$C_3H_7$ | O | $CH_2$-CH=$CH_2$ | $CH_3$ | 1.5028 |
| 163 | n-$C_3H_7$ | O | 1-Propin-yl-3 | $CH_3$ | 1.5100 |
| 164 | $C_2H_5$ | O | 2,6-Dichlorbenzyl | $CH_3$ | |
| 165 | $C_2H_5$ | S | Allyl | $CH_3$ | |
| 166 | $CH_3$ | O | Benzyl | $C_2H_5$ | 115-118 |
| 167 | $CH_3$ | O | 2-Chlorbenzyl | $CH_3$ | 91- 95 |
| 168 | $C_2H_5$ | O | 2-Chlorbenzyl | $CH_3$ | |
| 169 | $CH_3$ | O | 2-Chlorbenzyl | $C_2H_5$ | |
| 170 | $CH_3$ | O | 2,6-Dichlorbenzyl | $C_2H_5$ | |
| 171 | $C_2H_5$ | O | Benzyl | $C_2H_5$ | |
| 172 | $C_2H_5$ | O | 4-Nitrophenyl | $C_2H_5$ | |
| 173 | $CH_3$ | O | 3-Chlorbenzyl | $CH_3$ | 89- 92 |
| 174 | $C_2H_5$ | O | 3-Chlorbenzyl | $C_2H_5$ | |
| 175 | $CH_3$ | O | 3-Chlorbenzyl | $CH_3$ | |
| 176 | $CH_3$ | S | $CH_2$-CH=$CH_2$ | $CH_3$ | |

| Verbindung Nr. | $R^1$ | Y | $R^2$ | $R^3$ | Fp[°C]/$n_D^{25}$ |
|---|---|---|---|---|---|
| 177 | $CH_3$ | O | 2-Methyl-propen-1-yl-3 | $CH_3$ | 1.5051 |
| 178 | i-$C_3H_7$ | O | 4-Nitrophenyl | $CH_3$ | 159-163 |
| 179 | $CH_3$ | O | 3,4-Dichlorbenzyl | $CH_3$ | |
| 180 | $CH_3$ | O | 3,4-Dichlorbenzyl | $C_2H_5$ | |
| 181 | i-$C_4H_9$ | O | 3-Methoxybenzyl | $CH_3$ | 90- 94 |
| 182 | $C_2H_5$ | O | 3-Trifluormethyl-phenyl | H | |
| 183 | $C_2H_5$ | O | 2-Fluor-6-chlorbenzyl | $CH_3$ | |
| 184 | $CH_3$ | O | 4-Chlorbenzyl | $C_2H_5$ | |
| 185 | $CH_3$ | O | 3-Methoxybenzyl | $C_2H_5$ | 82- 85 |
| 186 | $CH_3$ | O | $CH_2$-$CCl_3$ | $CH_3$ | 71- 75 |
| 187 | $CH_3$ | O | $ClCH_2$-$(CH_2)_2$- | $CH_3$ | 1.5080 |
| 188 | $N(CH_3)_2$ | O | Allyl | $CH_3$ | |
| 189 | $N(CH_3)_2$ | O | Prop-1-in-3-yl | $CH_3$ | |
| 190 | $N(CH_3)_2$ | O | $CH_3$ | $CH_3$ | 159-162 |
| 191 | $N(CH_3)_2$ | O | Benzyl | $CH_3$ | 114-118 |
| 192 | $N(CH_3)_2$ | O | 4-Nitrophenyl | $CH_3$ | 202-204 |
| 193 | $CH_3$ | O | Cyclopentyl | $CH_3$ | |
| 194 | $CH_3$ | O | Cyclohexyl | $CH_3$ | |
| 195 | $CH_3$ | O | Methoxycarbonylmethyl | $CH_3$ | |
| 196 | $CH_3$ | O | Methylmercaptocarbonylmethyl | $CH_3$ | |
| 197 | $CH_3$ | O | 2-(2,4-Dichlorphenoxy)-ethyl | $CH_3$ | |
| 198 | $CH_3$ | O | 2-(3-Methoxyphenoxy)-ethyl | $CH_3$ | |

| Verbindung Nr. | $R^1$ | Y | $R^2$ | $R^3$ | Fp[°C]/$n_D^{25}$ |
|---|---|---|---|---|---|
| 199 | $N(C_2H_5)_2$ | O | $CH_2-C{\equiv}CH$ | $CH_3$ | 58-62 |
| 200 | $CH_3$ | O | 2,4-Dichlorbenzyl | $CH_3$ | 117-118 |
| 201 | $CH_3$ | O | $(CH_2)_4CH_3$ | $CH_3$ | 1.4860 |
| 202 | $CH_3$ | O | $CH_2-CH_2-O-CH_3$ | $CH_3$ | |
| 203 | $CH_3$ | O | $CH_2-CH_2-O-C_2H_5$ | $CH_3$ | |
| 204 | $CH_3$ | O | $CH_2-CH=C(CH_3)_2$ | $CH_3$ | |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spitzpulvern, Oldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung gemäß Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung gemäß Beispiel 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung gemäß Beispiel 4 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 35 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer

Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 13 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 36 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 46 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen oder während des Auflaufens der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr, vorzugsweise 0,5 bis 4,0 kg/ha, wobei sich die höheren Dosen besonders zur totalen Bekämpfung von Vegetationen eignen.

Die herbizide Wirkung von Verbindungen der Formel I wird durch Gewächshausversuche gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Dabei handelt es sich um entweder direkt in die zu behandelnden Gefäße gesäte Pflanzen oder um separat angezogene Pflanzen, die in die Gefäße umgepflanzt wurden. Die Abdeckung unterbleibt nach der Blattbehandlung.

Die Versuchsgefäße werden im Gewächshaus aufgestellt, wobei für wärmeliebende Arten wärmere Bereiche (20 bis 30 °C) und für solche gemäßigter Klimate 15 bis 25 °C bevorzugt werden. Die Versuchsperiode erstreckt sich über 3 bis 5 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Testpflanzen handelt es sich um

Amaranthus spp. (Fuchsschwanzarten),
Chenopodium album (Weißer Gänsefuß),
Chrysanthemum spp. (Wucherblume-Arten),
Datura stramonium (Stechapfel),
Galium aparine (Klettenlabkraut),
Gossypium hirsutum (Baumwolle),
Matricaria spp. (Kamillearten),
Nicandra physaloides (Giftbeere),
Oryza sativa (Reis),
Sesbania exaltata (Turibaum),
Sinapis alba (Weißer Senf),
Solanum nigrum (Schwarzer Nachtschatten),
Triticum aestivum (Weizen),
Zea mays (Mais),
Lolium multiflorum (Ital. Raygras),
Ipomoea spp. (Prunkwindearten),
Cyperus esculentus (Erdmandelgras),
Centaurea cyanus (Kornblume).

Die Versuche zeigen, daß beispielsweise die Verbindungen Nr. 2, 51, 109 und 154 bei Vorauflaufanwendung von 2,0 kg Wirstoff/ha eine gute herbizide Wirkung haben. Gleichzeitig werden einige Kulturpflanzen in selektiver Weise weitgehend geschont. Die Verbindung Nr. 186 bekämpft in geringer Aufwandmenge breitblättrige Unkräuter in Mais, während die Verbindung Nr. 47 unerwünschten Pflanzenwuchs in Reis bekämpft.

Die Verbindung Nr. 112 bekämpft im Nachauflaufverfahren mit einer geringeren Menge an Wirkstoff eine Reihe von unerwünschten Pflanzen. Die Verbindungen Nr. 179 und 187 zeigen sowohl im Vor- als auch im Nachauflaufverfahren eine gute herbizide Wirkung.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifoluim) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |

(Fortsetzung)

| Botanischer Name | Deutscher Name |
|---|---|
| Phaseolus lunatus | Mondbohne |
| Phaseolus mungo | Erdbohne |
| Phaseolus vulgaris | Buschbohen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht-phytotoxische Öle und Ölkonzentrate zugesetzt werden.

**Ansprüche**

1. 2H-1,2,4,6-Thiatriazin-1,1-dioxide der Formel

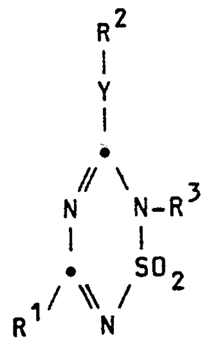

(I)

in der

$R^1$ Wasserstoff, einen gesättigten oder ungesättigten, unverzweigten oder verzweigten aliphatischen Rest mit bis zu 10 Kohlenstoffatomen, einen durch Halogen, Alkoxy oder Alkylmercapto mit 1 bis 4 Kohlenstoffatomen substituierten gesättigten, unverzweigten oder verzweigten aliphatischen Rest mit bis zu 10 Kohlenstoffatomen, einen Alkyl- oder Dialkylaminorest mit 1 bis 6 Kohlenstoffatomen in einer Alkylgruppe, einen Cycloalkylrest mit 3 bis 7 Kohlenstoffatomen, einen gegebenenfalls durch Halogen, Alkyl oder Alkoxy mit 1 bis 4 Kohlenstoffatomen substituierten Phenylrest oder einen gegebenenfalls durch Halogen substituierten Benzylrest,

$R^2$ einen gesättigten oder ungesättigten, unverzweigten oder verzweigten aliphatischen Rest mit bis zu 10 Kohlenstoffatomen, einen durch Halogen, Alkoxy oder Alkylmercapto mit 1 bis 4 Kohlenstoffatomen oder Alkanoyl mit 2 bis 8 Kohlenstoffatomen substituierten gesättigten oder ungesättigten, unverzweigten oder verzweigten aliphatischen Rest mit bis zu 10 Kohlenstoffatomen, einen Cycloalkylrest mit 3 bis 7 Kohlenstoffatomen, einen Alkoxycarbonylalkyl- oder Alkylmercaptocarbonylalkylrest mit jeweils 1 bis 4 Kohlenstoffatomen in einer Alkylgruppe, einen gegebenenfalls durch Halogen, einen Alkyl-, Halogenalkyl-, Alkoxy-, Halogenalkoxy-, Alkylmercapto-, Halogenalkylmercapto-, Alkylsulfinyl-, Halogenalkylsulfinyl-, Alkylsulfonyl- oder Halogenalkylsulfonylrest mit 1 bis 4 Kohlenstoffatomen, Nitro, Cyano, Azido, Carboxy, Thiocyanato, Hydroxyiminomethyl, Formyl, einen Alkoxycarbonyl- oder Alkylmercaptocarbonylrest mit 2 bis 5 Kohlenstoffatomen, einen N-Alkylcarbamoyl-, N,N-Dialkylcarbamoyl-, N-Alkylcarbamoylamino- oder N,N-Dialkylcarbamoylaminorest mit 1 bis 4 Kohlenstoffatomen in einer Alkylgruppe, einen 4-Alkyl-1,2,4-oxadiazolidin-3,5-dion-2-ylrest, einen Alkanoylamino-, Alkoxycarbonylamino- oder Alkylmercaptocarbonyl- aminorest mit 2 bis 5 Kohlenstoffatomen, einen N-Alkylsulfamoyl, N,N-Dialkylsulfamoyl-, N-Alkylsulfamoylamino- oder N,N-Dialkylsulfamoylaminorest mit 1 bis 4 Kohlenstoffatomen in einer Alkylgruppe, einen Alkoxysulfonylrest mit 1 bis 4 Kohlenstoffatomen, einen Alkoxycarbonylaminosulfonylrest mit 2 bis 5 Kohlenstoffatomen oder einen Alkoxycarbonylalkoxy-, Alkylmercaptocarbonylalkoxy-, Alkoxycarbonylalkylmercapto- oder Alkymercaptocarbonylalkylmercaptorest mit jeweils 1 bis 4 Kohlenstoffatomen in einer Alkylgruppe substituierten Phenylrest, einen gegebenenfalls durch Halogen, Nitro, einen Alkyl-, Halogenalkyl-, Alkoxy-, Halogenalkoxy- oder Halogenalkylmercaptorest mit 1 bis 4 Kohlenstoffatomen substituierten Benzylrest oder einen gegebenenfalls durch Halogen, einen Alkyl-, Alkoxy-, Halogenalkyl-, Halogenalkoxy- oder Halogenalkylmercapto mit 1 bis 4 Kohlenstoffatomen am Phenylring substituierten Phenoxyalkylrest mit 2 bis 4 Kohlenstoffatomen in der Alkylgruppe,

$R^3$ Wasserstoff, einen gesättigten oder ungesättigten, unverzweigten oder verzweigten aliphatischen Rest mit bis zu 10 Kohlenstoffatomen, einen durch Halogen oder Alkoxy mit 1 bis 4 Kohlenstoffatomen substituierten gesättigten, unverzweigten oder verzweigten aliphatischen Rest mit bis zu 10 Kohlenstoffatomen oder einen Cycloalkylrest mit 3 bis 7 Kohlenstoffatomen und

Y Sauerstoff, Schwefel, —SO— oder —$SO_2$— bedeuten.

2. 2H-1,2,4,6-Thiatriazin-1,1-dioxid der Formel I gemäß Anspruch 1, dadurch gekennzeichnet, daß $R^1$, $R^2$ und $R^3$ für Alkyl mit 1 bis 4 Kohlenstoffatomen und Y für Sauerstoff oder Schwefel stehen.

3. 2H-1,2,4,6-Thiatriazin-1,1-dioxid der Formel I gemäß Anspruch 1, dadurch gekennzeichnet, daß $R^1$ und $R^3$ für Alkyl mit 1 bis 4 Kohlenstoffatomen, $R^2$ für einen ungesättigten aliphatischen Rest mit 3 oder 4 Kohlenstoffatomen und Y für Sauerstoff oder Schwefel stehen.

4. 2H-1,2,4,6-Thiatriazin-1,1-dioxid der Formel I gemäß Anspruch 1, dadurch gekennzeichnet, daß $R^1$ und $R^3$ für Alkyl mit 1 bis 4 Kohlenstoffatomen, $R^2$ für einen durch Halogen, Nitro, Cyano oder Alkyl mit 1 bis 4 Kohlenstoffatomen substituierten Phenylrest und Y für Sauerstoff oder Schwefel stehen.

5. 2H-1,2,4,6-Thiatriazin-1,1-dioxid der Formel I gemäß Anspruch 1, dadurch gekennzeichnet, daß $R^1$ und $R^3$ für Alkyl mit 1 bis 4 Kohlenstoffatomen, $R^2$ für einen gegebenenfalls durch Halogen, einen Alkyl-, Alkoxy- oder Halogenalkoxyrest mit 1 bis 4 Kohlenstoffatomen substituierten Benzylrest und Y für Sauerstoff oder Schwefel stehen.

6. Verfahren zur Herstellung von 2H-1,2,4,6-Thiatriazin-1,1-dioxiden der Formel I gemäß Anspruch 1, dadurch gekennzeichnet, daß man ein substituiertes 2H-1,2,4,6-Thiatriazin-1,1-dioxid der Formel

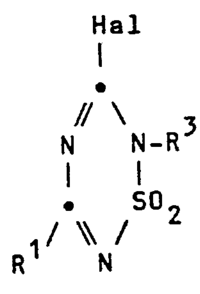

(II)

in der $R^1$ und $R^3$ die im Anspruch 1 genannten Bedeutungen haben und Hal für Halogen steht, mit einer Verbindung der Formel

$$H—Y—R^2 \quad (III),$$

in der $R^2$ und Y die im Anspruch 1 genannten Bedeutungen haben, oder einem Alkali-, Erdalkali- oder Ammoniumsalz einer Verbindung der Formel III, gegebenenfalls in einem inerten organischen Lösungsmittel und gegebenenfalls in Anwesenheit eines Säureakzeptors umsetzt.

7. Herbizid, enthaltend inerte Zusatzstoffe und ein 2H-1,2,4,6-Thiatriazin-1,1-dioxid der Formel

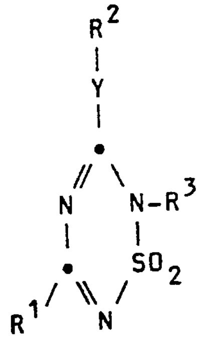

(I)

in der

$R^1$ Wasserstoff, einen gesättigten oder ungesättigten, unverzweigten oder verzweigten aliphatischen Rest mit bis zu 10 Kohlenstoffatomen, einen durch Halogen, Alkoxy oder Alkylmercapto mit 1 bis 4 Kohlenstoffatomen substituierten gesättigten, unverzweigten oder verzweigten aliphatischen Rest mit bis zu 10 Kohlenstoffatomen, einen Alkyl- oder Dialkylaminorest mit 1 bis 6 Kohlenstoffatomen in einer Alkylgruppe, einen Cycloalkylrest mit 3 bis 7 Kohlenstoffatomen, einen gegebenenfalls durch Halogen, Alkyl oder Alkoxy mit 1 bis 4 Kohlenstoffatomen substituierten Phenylrest oder einen gegebenenfalls durch Halogen substituierten Benzylrest,

$R^2$ einen gesättigten oder ungesättigten, unverzweigten oder verzweigten aliphatischen Rest mit bis zu 10 Kohlenstoffatomen, einen durch Halogen, Alkoxy oder Alkylmercapto mit 1 bis 4 Kohlenstoffatomen oder Alkanoyl mit 2 bis 8 Kohlenstoffatomen substituierten gesättigten oder ungesättigten, unverzweigten oder verzweigten aliphatischen Rest mit bis zu 10 Kohlenstoffatomen, einen Cycloalkylrest mit 3 bis 7 Kohlenstoffatomen, einen Alkoxycarbonylalkyl- oder Alkylmercaptocarbonylalkylrest mit jeweils 1 bis 4 Kohlenstoffatomen in einer Alkylgruppe, einen gegebenenfalls durch Halogen, einen Alkyl-, Halogenalkyl-, Alkoxy-, Halogenalkoxy-, Alkylmercapto-, Halogenalkylmercapto-, Alkylsulfinyl-, Halogenalkylsulfinyl-, Alkylsulfonyl- oder Halogenalkylsulfonylrest mit 1 bis 4 Kohlenstoffatomen, Nitro, Cyano, Azido, Carboxy, Thiocyanato, Hydroxyiminomethyl, Formyl, einen Alkoxycarbonyl- oder Alkylmercaptocarbonylrest mit 2 bis 5 Kohlenstoffatomen, einen N-Alkylcarbamoyl-, N,N-Dialkylcarbamoyl-, N-Alkylcarbamoylamino- oder N,N-Dialkylcarbamoylaminorest mit 1 bis 4 Kohlenstoffatomen in einer Alkylgruppe, einen 4-Alkyl-1,2,4-oxadiazolidin-3,5-dion-2-ylrest, einen Alkanoylamino-, Alkoxycarbonylamino- oder Alkylmercaptocarbonylaminorest mit 2 bis 5 Kohlenstoffatomen, einen N-Alkylsulfamoyl-, N,N-Dialkylsulfamoyl-, N-Alkylsulfamoylamino- oder N,N-Dialkylsulfamoylaminorest mit 1 bis 4 Kohlenstoffatomen in einer Alkylgruppe, einen Alkoxysulfonylrest mit 1 bis 4 Kohlenstoffatomen, einen Alkoxycarbonylaminosulfonylrest mit 2 bis 5 Kohlenstoffatomen oder einen Alkoxycarbonylalkoxy-, Alkylmercaptocarbonylalkoxy-, Alkoxycarbonylalkylmercapto- oder Alkylmercaptocarbonylalkylmercaptorest mit jeweils 1 bis 4 Kohlenstoffatomen in einer Alkylgruppe substituierten Phenylrest, einen gegebenenfalls durch Halogen, Nitro, einen Alkyl-, Halogenalkyl-, Alkoxy-, Halogenalkoxy- oder Halogenalkylmercaptorest mit 1 bis 4 Kohlenstoffatomen substituierten Benzylrest oder einen gegebenenfalls durch Halogen, einen Alkyl-, Alkoxy-, Halogenalkyl-, Halogenalkoxy- oder Halogenalkylmercapto mit 1 bis 4 Kohlenstoffatomen am Phenylring substituierten Phenoxyalkylrest mit 2 bis 4 Kohlenstoffatomen in der Alkylgruppe,

$R^3$ Wasserstoff, einen gesättigten oder ungesättigten, unverzweigten oder verzweigten aliphatischen Rest mit bis zu 10 Kohlenstoffatomen, einen durch Halogen oder Alkoxy mit 1 bis 4 Kohlenstoffatomen substituierten gesättigten, unverzweigten oder verzweigten aliphatischen Rest mit bis zu 10 Kohlenstoffatomen oder einen Cycloalkylrest mit 3 bis 7 Kohlenstoffatomen und Sauerstoff, Schwefel, —SO— oder —$SO_2$— bedeuten, als Wirkstoff.

8. Herbizid nach Anspruch 7, dadurch gekennzeichnet, daß $R^1$ und $R^3$ für Alkyl mit 1 bis 4 Kohlenstoffatomen, $R^2$ für einen ungesättigten aliphatischen Rest mit 3 oder 4 Kohlenstoffatomen und Y für Sauerstoff oder Schwefel stehen.

9. Herbizid nach Anspruch 7, dadurch gekennzeichnet, daß $R^1$ und $R^3$ für Alkyl mit 1 bis 4 Kohlenstoffatomen, $R^2$ für einen durch Halogen, Nitro, Cyano oder Alkyl mit 1 bis 4 Kohlenstoffatomen substituierten Phenylrest und Y für Sauerstoff oder Schwefel stehen.

10. Verfahren zur Bekämpfung unerwünschten Pflanzenwuchses, dadurch gekennzeichnet, daß man die Pflanzen und/oder ihren Standort mit einer herbizid wirksamen Menge eines 2H-1,2,4,6-Thiatriazin-1,1-dioxids der Formel I gemäß Anspruch 1 behandelt.

**Claims**

1. A 2H-1,2,4,6-thiatriazine-1,1-dioxide of the formula

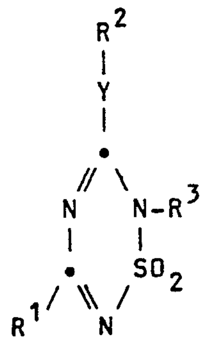

(I)

where

$R^1$ is hydrogen, a saturated or unsaturated straight-chain or branched aliphatic radical of not more than 10 carbon atoms, a saturated straight-chain or branched aliphatic radical of not more than 10 carbon atoms which is substituted by halogen, alkoxy or alkylmercapto of 1 to 4 carbon atoms, an alkyl or dialkylamino radical where alkyl is of 1 to 6 carbon atoms, cycloalkyl of 3 to 7 carbon atoms, phenyl which is unsubstituted or substituted by halogen, alkyl or alkoxy of 1 to 4 carbon atoms, or benzyl which is unsubstituted or substituted by halogen,

$R^2$ is a saturated or unsaturated straight-chain or branched aliphatic radical of not more than 10 carbon atoms, a saturated or unsaturated straight-chain or branched aliphatic radical of not more than 10 carbon atoms which is substituted by halogen, alkoxy or alkylmercapto of 1 to 4 carbon atoms or alkanoyl of 2 to 8 carbon atoms, cycloalkyl of 3 to 7 carbon atoms, alkoxycarbonylalkyl or alkylmercaptocarbonylalkyl where each alkyl is of 1 to 4 carbon atoms, phenyl which is unsubstituted or substituted by halogen, an alkyl, haloalkyl, alkoxy, haloalkoxy, alkylmercapto, haloalkylmercapto, alkylsulfinyl, haloalkylsulfinyl, alkylsulfonyl or haloalkylsulfonyl radical of 1 to 4 carbon atoms, nitro, cyano, azido, carboxyl, thiocyanato, hydroxyiminomethyl, formyl, an alkoxycarbonyl or alkylmercaptocarbonyl radical of 2 to 5 carbon atoms, an N-alkylcarbamyl, N,N-dialkylcarbamyl, N-alkylcarbamylamino or N,N-dialkylcarbamylamino radical where alkyl is of 1 to 4 carbon atoms, 4-alkyl-1,2,4-oxadiazolidine-3,5-dion-2-yl, an alkanoylamino, akoxycarbonylamino or alkylmercaptocarbonylamino radical of 2 to 5 carbon atoms, an N-alkylsulfamyl, N,N-dialkysulfamyl, N-alkylsulfamylamino or N,N-dialkylsulfamylamino radical where alkyl is of 1 to 4 carbon atoms, alkoxysulfonyl of 1 to 4 carbon atoms, alkoxycarbonylaminosulfonyl of 2 to 5 carbon atoms or an alkoxycarbonylalkoxy, alkylmercaptocarbonylalkoxy, alkoxycarbonylalkylmercapto or alkylmercaptocarbonylalkylmercapto radical where each alkyl is of 1 to 4 carbon atoms, or $R^2$ is benzyl which is unsubstituted or substituted by halogen, nitro or an alkyl, haloalkyl, alkoxy, haloalkoxy or haloalkylmercapto radical of 1 to 4 carbon atoms, or phenoxyalkyl where alkyl is of 2 to 4 carbon atoms, which is unsubstituted or substituted in the phenyl ring by halogen or by alkyl, alkoxy, haloalkyl, haloalkoxy or haloalkylmercapto of 1 to 4 carbon atoms,

$R^3$ is hydrogen, a satured or unsaturated straight-chain or branched aliphatic radical of not more than 10 carbon atoms, or a saturated straight-chain or branched aliphatic radical of not more than 10 carbon atoms which is substituted by halogen or by alkoxy of 1 to 4 carbon atoms, or is cycloalkyl of 3 to 7 carbon atoms, and

Y is oxygen, sulfur, —SO— or —$SO_2$.

2. A 2H-1,2,4,6-thiatriazine-1,1dioxide of the formula I as claimed in claim 1, where $R^1$, $R^2$ and $R^3$ are alkyl of 1 to 4 carbon atoms and Y is hydrogen or sulfur.

3. A 2H-1,2,4,6-thiatriazine-1,1-dioxide of the formula I as claimed in claim 1, where $R^1$ and $R^3$ are alkyl of 1 to 4 carbon atoms, $R^2$ is an unsaturated aliphatic radical of 3 or 4 carbon atoms, and Y is oxygen or sulfur.

4. A 2H-1,2,4,6-thiatriazine-1,1dioxide of the formula I as claimed in claim 1, where $R^1$ and $R^3$ are alkyl of 1 to 4 carbon atoms, $R^2$ is phenyl substituted by halogen, nitro, cyano or alkyl of 1 to 4 carbon atoms, and Y is oxygen or sulfur.

5. A 2H-1,2,4,6-thiatriazine-1,1dioxide of the formula I as claimed in claim 1, where $R^1$ and $R^3$ are alkyl of 1 to 4 carbon atoms, $R^2$ is benzyl which is unsubstituted or substituted by halogen, or by alkyl, alkoxy or haloalkoxy of 1 to 4 carbon atoms, and Y is oxygen or sulfur.

6. A process for manufacturing 2H-1,2,4,6-thiatriazine-1,1-dioxides of the formula I as claimed in claim 1, wherein a substituted 2H-1,2,4,6-thiatriazine-1,1-dioxide of the formula

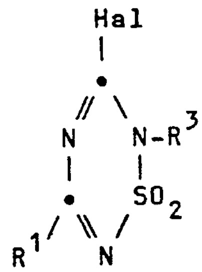

(II)

where $R^1$ and $R^3$ have the meanings given in claim 1 and Hal is halogen, is reacted with a compound of the formula

H—Y—$R^2$ (III)

where $R^2$ and Y have the meanings given in claim 1, or with an alkali metal salt, alkaline earth metal salt or ammonium salt of a compound of the formula III, if necessary in an inert organic solvent, and in the presence or absence of an acid acceptor

7. A herbicide containing inert additives and, as active ingredient, a 2H-1,2,4,6-thiatriazine-1, 1-dioxide of the formula

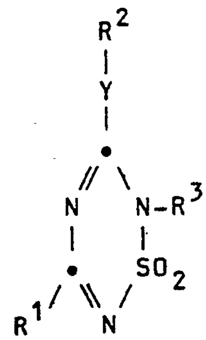

(I)

where

$R^1$ is hydrogen, a saturated or unsaturated straight-chain or branched aliphatic radical of not more than 10 carbon atoms, a saturated straight-chain or branched aliphatic radical of not more than 10 carbon atoms which is substituted by halogen, alkoxy or alkylmercapto of 1 to 4 carbon atoms, an alkyl or dialkylamino radical where alkyl is of 1 to 6 carbon atoms, cycloalkyl of 3 to 7 carbon atoms, phenyl which is unsubstituted or substituted by halogen, alkyl or alkoxy of 1 to 4 carbon atomes, or benzyl which is unsubstituted or substituted by halogen,

$R^2$ is a saturated or unsaturated straight-chain or branched aliphatic radical of not more than 10 carbon atoms, a saturated or unsaturated straight-chain or branched aliphatic radical of not more than 10 carbon atoms which is substituted by halogen, alkoxy or alkylmercapto of 1 to 4 carbon atoms or alkanoyl of 2 to 8 carbon atoms, cycloalkyl of 3 to 7 carbon atoms, alkoxycarbonylalkyl or alkylmercaptocarbonylalkyl where each alkyl is of 1 to 4 carbon atoms, phenyl which is unsubstituted or substituted by halogen, an alkyl, halogalkyl, alkoxy, haloalhoxy, alkylmercapto, haloalkylmercapto, alkylsulfinyl, haloalkysulfinyl, alkylsulfonyl or haloalkylsulfonyl radical of 1 to 4 carbon atoms, nitro, cyano, azido, carboxyl, thiocyanato, hydroxyiminomethyl, formyl, an alkoxycarbonyl or alkylmercaptocarbonyl radical of 2 to 5 carbon atoms, an N-alkylcarbamyl, N,N-diakylcarbamyl, N-alkylcarbamylamino or N-N-dialkylcarbamylamino radical where alkyl is of 1 to 4 carbon atoms, 4-alkyl-1,2,4-oxadiazolidine-3,5-dion-2-yl, an alkanoylamino, akoxycarbonylamino or alkylmercaptocarbonylamino radical of 2 to 5 carbon atoms, an N-alkylsulfamyl, N,N-dialkylsulfamyl, N-alkylsulfamylamino or N,N-dialkylsulfamylamino radical where alkyl is of 1 to 4 carbon atoms, alkoxysulfonyl of 1 to 4 carbon atoms, alkoxycarbonylaminosulfonyl of 2 to 5 carbon atoms or atoms alkoxycarbonylalkoxy, alkylmercaptocarbonylalkoxy, alkoxycarbonylalkylmercapto or alkylmercaptocarbonylalkylmercapto radical where each alkyl is of 1 to 4 carbon atoms, or $R^2$ is benzyl which is unsubsistuted or susbituted by halogen, nitro or an alkyl, haloalkyl, alkoxy, haloalkoxy or haloalkylmercapto radical of 1 to 4 carbon atoms, or phenoxyalkyll where alkyl is of 2 to 4 carbon atoms, which is unsubstituted or substituted in the phenyl ring by halogen or by alkyl, alkoxy, haloalkyl, haloalkoxy or haloalkylmercapto of 1 to 4 carbon atoms,

$R^3$ is hydrogen, a saturated or unsaturated straight-chain or branched aliphatic radical of not more than 10 carbon atoms, or a saturated straight-chain or branched aliphatic radical of not more than 10 carbon atoms which is substituted by halogen or by alkoxy of 1 to 4 carbon atoms, or is cycloalkyl of 3 to 7 carbon atoms, and

Y is oxygen, sulfur, —SO— or —$SO_2$—.

8. A herbicide as claimed in claim 7, where $R^1$ and $R^3$ are alkyl of 1 to 4 carbon atoms, $R^2$ is an unsaturated aliphatic radical of 3 or 4 carbon atoms, and Y is oxygen or sulfur.

9. A herbicide as claimed in claim 7, where $R^1$ and $R^3$ are alkyl of 1 to 4 carbon atoms, $R^2$ is phenyl substituted by halogen, nitro, cyano or alkyl of 1 to 4 carbon atoms, and Y is oxygen or sulfur.

10. A process for combating the growth of unwanted plants, wherein the plants and/or their location are treated with a herbicidally effective amount of a 2H-1,2,4,6-thiatriazine-1,1-dioxide of the formula I as claimed in claim 1.

**Revendications**

1. 2H-1,2,4,6-Thiatriazine-1,1-dioxydes de la formule

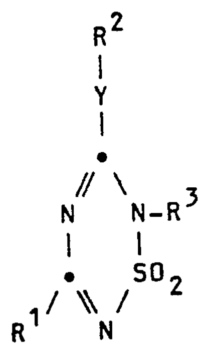

(I)

dans laquelle

$R^1$ désigne un atome d'hydrogène, un groupe aliphatique ramifié ou non, saturé ou non, contenant jusqu'à 10 atomes de carbone, un groupe aliphatique saturé, ramifié ou non, contenant jusqu'à 10 atomes de carbone, halo-, alcoxy- ou alkyl (en $C_1$ à $C_4$)-mercapto-substitué, un groupe alkyl- ou dialkyl-amino à radicaux alkyle en $C_1$ à $C_6$, un groupe cycloalkyle en $C_3$ à $C_7$, un groupe phényle éventuellement halo-, alkyl ou alcoxy (en $C_1$ à $C_4$)-substitué ou un groupe benzyle éventuellement halo-substitué ;

$R^2$ représente un groupe aliphatique ramifié ou non, saturé ou non, contenant jusqu'à 10 atomes de carbone, un groupe aliphatique ramifié ou non, saturé ou non, avec jusqu'à 10 atomes de carbone, halo-, alcoxy-, alkyl (en $C_1$ à $C_4$)-mercapto- ou alcanoyl (en $C_2$ à $C_8$)-substitué, un groupe cycloalkyle en $C_3$ à $C_7$, un groupe alcoxy-carbonyl-alkyle ou alkyl-mercapto-carbonyl-alkyle à radicaux alkyle en $C_1$ à $C_4$, un groupe phényle éventuellement substitué par un halogène ou par un groupe alkyle, halo-alkyle, alcoxy, halo-alkoxy, alkyl-mercapto, halo-alkyl-mercapto, alkyl-sulfinyle, halo-alkyl-sulfinyle, alkyl-sulfonyle ou halo-alkyl-sulfonyle en $C_1$ à $C_4$, par un groupe nitro, cyano, azido, carboxy, thiocyanato, hydroxy-imino-méthyle ou formyle, par un groupe alcoxy-carbonyle ou alkyl-mercapto-carbonyle en $C_2$ à $C_5$, par un groupe N-alkyl-carbamoyle, N,N-dialkyl-carbamoyle, N-alkyl-carbamoyl-amino ou N,N-dialkyl-carbamoyl-amino à radicaux alkyle en $C_1$ à $C_4$, par un groupe 4-alkyl-1,2,4-oxadiazolidine-3,5-dione-2-yle, par un groupe alcanoyl-amino, alcoxy-carbonyl-amino ou alkyl-mercapto-carbonyl-amino en $C_2$ à $C_5$, par un groupe N-alkyl-sulfamoyle, N,N-dialkyl-sulfamoyle, N-alkyl-sulfamoyl-amino ou N,N-dialkyl-sulfamoyl-amino à radicaux alkyle en $C_1$ à $C_4$ ou par un groupe alcoxy-sulfonyle en $C_1$ à $C_4$, alcoxy-carbonylamino-sulfonyle en $C_2$ à $C_5$, alcoxy-carbonyl-alcoxy, alkyl-mercapto-carbonyl-alcoxy, alcoxy-carbonylalkyl-mercapto ou alkyl-mercapto-carbonyl-alkyl-mercapto à radicaux alkyle en $C_1$ à $C_4$, un groupe benzyle éventuellement substitué par un halogène ou un groupe nitro, alkyle, halo-alkyle, alcoxy, haloalcoxy ou halo-alkyl-mercapto en $C_1$ à $C_4$ ou un groupe phénoxy-alkyle à radicaux alkyle en $C_2$ à $C_4$, éventuellement substitué sur le noyau phényle par un halogène ou un groupe alkyle, alcoxy, halo-alkyle, halo-alcoxy ou halo-alkyl-mercapto en $C_1$ à $C_4$ ;

$R^3$ désigne un atome d'hydrogène, un groupe aliphatique ramifié ou non, saturé ou non, contenant jusqu'à 10 atomes de carbone, éventuellement halo- ou alcoxy (en $C_1$ à $C_4$) substitué, ou un groupe cycloalkyle en $C_3$ à $C_7$ et

X désigne un atome d'oxygène ou de soufre ou un groupe —SO ou —$SO_2$—.

2. 2H-1,2,4,6-Thiatriazine-1,1-dioxyde de la formule I selon la revendication 1, caractérisé en ce que $R^1$, $R^2$ et $R^3$ représentent des radicaux alkyle en $C_1$ à $C_4$ et

Y est de l'oxygène ou du soufre.

3. 2H-1,2,4,6-thiatriazine-1,1-dioxyde de la formule 1 selon la revendication 1, caractérisé en ce que $R^1$ et $R^3$ représentent des radicaux alkyle en $C_1$ à $C_4$, $R^2$ désigne un groupe aliphatique non saturé en $C_3$ ou $C_4$ et

Y est de l'oxygène ou du soufre.

4. 2H-1,2,4,6-Thiatrizine-1,1-dioxyde de la formule I selon la revendication 1, caractérisé en ce que $R^1$ et $R^3$ représentent des radicaux alkyle en $C_1$ à $C_4$, $R^2$ désigne un groupe phényle halo-, nitro, cyano- ou alkyl (en $C_1$ à $C_4$)-substitué et Y est un atome d'oxygène ou de soufre.

5. 2H-1,2,4,6-Thiatriazine-1,1-dioxyde de la formule I selon la revendication 1, caractérisé en ce que $R^1$ et $R^3$ représentent des radicaux alkyle en $C_1$ à $C_4$, $R^2$ désigne un groupe benzyle éventuellement substitué par un halogène ou un groupe alkyle, alcoxy ou halo-alcoxy en $C_1$ à $C_4$ et Y est de l'oxygène ou du soufre.

6. Procédé de préparation de 2H-1,2,4,6-thiatriazine-1,1-dioxydes de la formule I selon la revendication 1, caractérisé en ce que l'on fait réagir, le cas échéant dans un solvant organique inerte et éventuellement en présence d'un fixateur d'acide, un 2H-1,2,4,6-thiatriazine-1,1-dioxide substitué de la formule

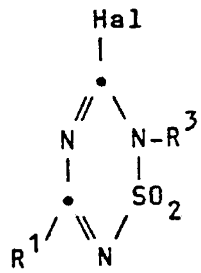

(II)

dans laquelle $R^1$ et $R^3$ possèdent les significations définies dans la revendication 1 et Hal désigne un atome d'halogène, avec un composé de la formule

$$H—Y—R^2 \qquad (III),$$

dans laquelle $R^2$ et Y possèdent les significations définies dans la revendication 1, ou avec un sel de métal alcalin ou de métal alcalino-terreux ou d'ammonium d'un composé de la formule III.

7. Composition herbicide, contenant des additifs inertes et en tant que principe actif un 2H-1,2,4,6-thiatriazine-1,1-dioxyde de la formule

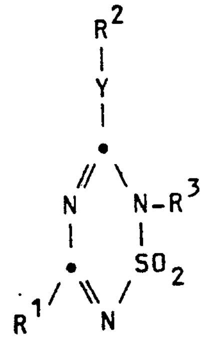

(I)

dans laquelle

$R^1$ désigne un atome d'hydrogène, un groupe aliphatique ramifié ou non, saturé ou non, contenant jusqu'à 10 atomes de carbone, un groupe aliphatique saturé, ramifié ou non, contenant jusqu'à 10 atomes de carbone, halo-, alcoxy- ou alkyl (en $C_1$ à $C_4$)-mercapto-substitué, un groupe alkyl- ou dialkyl-amino à radicaux alkyle en $C_1$ à $C_6$, un groupe cycloalkyle en $C_3$ à $C_7$, un groupe phényle éventuellement halo-, alkyl- ou alcoxy (en $C_1$ à $C_4$)-substitué ou un groupe benzyle éventuellement halo-substitué ;

$R^2$ représente un groupe aliphatique ramifié ou non, saturé ou non, contenant jusqu'à 10 atomes de carbone, un groupe aliphatique ramifié ou non, saturé ou non, avec jusqu'en 10 atomes de carbone, halo-, alcoxy-, alkyl (en $C_1$ à $C_4$)-mercapto- ou alcanoyl (en $C_2$ à $C_8$)-substitué, un groupe cycloalkyle en $C_3$ à $C_7$, un groupe alcoxy-carbonyl-alkyle ou alkyl-mercapto-carbonyl-alkyle à radicaux alkyle en $C_1$ à $C_4$, un groupe phényle éventuellement substitué par un halogène ou par un groupe alkyle, halo-alkyle, alcoxy, halo-alcoxy, alkyl-mercapto, halo-alkyl-mercapto, alkyl-sulfinyle, halo-alkyl-sulfinyle, alkyl-sulfonyle ou halo-alkyl-sulfonyle en $C_1$ à $C_4$, par un groupe nitro, cyano, azido, carboxy, thiocyanato, hydroxy-imino-méthyle ou formyle, par un groupe alcoxy-carbonyle ou alkyl-mercapto-carbonyle en $C_2$ à $C_5$, par un groupe N-alkyl-carbamoyle, N,N-dialkyl-carbamoyle, N-alkyl-carbamoyl-amino ou N,N-dialkyl-carbamoyl-amino à radicaux alkyle en $C_1$ à $C_4$, par un groupe 4-alkyl-1,2,4-oxadiazolidine-3,5-dione-2-yle, par un groupe alcanoyl-amino, alcoxy-carbonylamino ou alkyl-mercapto-carbonyl-amino en $C_2$ à $C_5$, par un groupe N-alkyl-sulfamoyle, N,N-dialkyl-sulfamoyle, N-alkyl-sulfamoyl-amino ou N,N-dialkyl-sulfamoyl-amino à radicaux alkyle en $C_1$ à $C_4$ ou par un groupe alcoxy-sulfonyle en $C_1$ à $C_4$, alcoxy-carbonylamino-sulfonyle en $C_2$ à $C_5$, alcoxy-carbonyl-alcoxy, alkyl-mercapto-carbonyl-alcoxy, alcoxy-carbonyl-alkyl-mercapto ou alkyl-mercapto-carbonyl-alkyl-mercapto à radicaux alkyle en $C_1$ à $C_4$, un groupe benzyle éventuellement substitué par un halogène ou un groupe nitro, alkyle, halo-alkyle, alcoxy, halo-alcoxy ou halo-alkyl-mercapto en $C_1$ à $C_4$ ou un groupe phénoxy-alkyle à radicaux alkyle en $C_2$ à $C_4$,

éventuellement substitué sur le noyau phényle par un halogène ou un groupe alkyle, alcoxy, halo-alkyle, halo-alcoxy ou halo-alkyl-mercapto en $C_1$ à $C_4$ ;

$R^3$ désigne un atome d'hydrogène, un groupe aliphatique ramifié ou non, saturé ou non, contenant jusqu'à 10 atomes de carbone, éventuellement halo- ou alcoxy (en $C_1$ à $C_4$) substitué, ou un groupe cycloalkyle en $C_3$ à $C_7$ et

X désigne un atome d'oxygène ou de soufre ou un groupe —SO ou —$SO_2$—.

8. Composition herbicide selon la revendication 7, caractérisé en ce que $R^1$ et $R^3$ représentent des radicaux alkyle en $C_1$ à $C_4$, $R^2$ désigne un groupe aliphatique non saturé en $C_3$ ou $C_4$ et Y est de l'oxygène ou du soufre.

9. Composition herbicide selon la revendication 7, caractérisé en ce que $R^1$ et $R^3$ représentent des radicaux alkyle en $C_1$ à $C_4$, $R^2$ désigne un groupe phényle halo-nitro, cyano- ou alkyl (en $C_1$ à $C_4$)-substitué et Y est de l'oxygène ou du soufre.

10. Procédé pour lutter contre la croissance de plantes indésirables, caractérisé en ce que l'on traite les plantes et(ou) leur biotope avec une quantité efficace du point de vue herbicide d'un 2H-1,2,4,6-thiatriazine-1,1-dioxyde de la formule I selon la revendication 1.